# EUROPÄISCHE PATENTSCHRIFT

(11) **EP 3 131 565 B1**
(45) Veröffentlichungstag und Bekanntmachung des Hinweises auf die Patenterteilung: **20.03.2019**
(21) Anmeldenummer: 15759930.9
(22) Anmeldetag: 17.04.2015
(51) Int. Cl.: A61K 36/899, A61K 36/185, A61P 1/00, A61P 17/00, A61P 17/02, A61P 17/06, A61P 3/10, A61K 8/97

(54) **PRÄPARAT AUS HAFERMEHL UND VERFAHREN ZU SEINER VERWENDUNG**
PREPARATION CONSISTING OF OATMEAL, AND A METHOD FOR THE USE OF SAME
PRÉPARATION COMPOSÉE DE FARINE D'AVOINE ET PROCÉDÉ D'UTILISATION DE CELLE-CI

(30) Priorität: 18.04.2014 EP 14165297; 16.09.2014 EP 14184897
(43) Veröffentlichungstag der Anmeldung: 22.02.2017
(73) Patentinhaber: Caricol - Digestive & Immune Health GmbH, 2392 Wienerwald/Grub (AT)
(72) Erfinder: CHOI, Danette, Vanessa, Mountain View, HI 96771 (US)
(74) Vertreter: Sonn & Partner Patentanwälte
(86) Internationale Anmeldenummer: PCT/IB2015/001205
(87) Internationale Veröffentlichungsnummer: WO 2015/159157

(56) Entgegenhaltungen:
- WO-A1-2004/047850
- WO-A2-2006/069390
- US-A1- 2009 311 376
- DATABASE WPI Week 201246 Thomson Scientific, London, GB; AN 2012-E61573 XP002727687, & CN 102 406 042 A (ZHANG X) 11. April 2012 (2012-04-11)
- DATABASE WPI Week 201433 Thomson Scientific, London, GB; AN 2014-F11525 XP002727688, & CN 103 519 078 A (HEFEI XIANGKOUFU IND & TRADE CO LTD) 22. Januar 2014 (2014-01-22)
- DATABASE WPI Week 201257 Thomson Scientific, London, GB; AN 2012-K50724 XP002727689, & CN 102 524 640 A (HEKANG TRADE SHANGHAI CO LTD) 4. Juli 2012 (2012-07-04)
- DATABASE WPI Week 201429 Thomson Scientific, London, GB; AN 2014-D65053 XP002727690, & CN 103 461 439 A (DANGTU RUILONG FRUIT TREE PLANTING) 25. Dezember 2013 (2013-12-25)
- MOONYEON YOUN ET AL: "Whole grain consumption has a modest effect on the development of diabetes in the Goto-Kakisaki rat", BRITISH JOURNAL OF NUTRITION, Bd. 107, Nr. 02, 30. Juni 2011 (2011-06-30), Seiten 192-201, XP055131393, UK ISSN: 0007-1145, DOI: 10.1017/S0007114511002741

## Beschreibung

Die Erfindung betrifft ein Präparat aus Naturprodukten, insbesondere zur Anwendung in der Behandlung von Hautkrankheiten. Ferner betrifft sie kosmetische Verfahren, umfassend die Anwendung des Präparats, und Verfahren zur Herstellung des Präparats.

Epidemiologischen Studien zu Folge leiden weltweit viele Patienten unter empfindlicher Haut. Auch Störungen wie Hauttrockenheit (Xerodermie) und Dermatitis, einschließlich Ekzeme, sind häufig. Ebenso Juckreiz, Rötung, rissige, offene, nässende, oder verdickte Haut. Als Dermatitis wird eine entzündliche Reaktion der Haut bezeichnet, die hauptsächlich die Dermis (Lederhaut) erfasst. Auch Schuppenflechte (Psoriasis) steht mit chronischen Entzündungsprozessen im Zusammenhang. Sonnenbrand und Insektenstiche dagegen stehen mit akuten entzündlichen Prozessen im Zusammenhang.

Auch Schleimhäute können Störungen oder Krankheiten unterliegen, die mit entzündlichen Prozessen im Zusammenhang stehen oder ein solcher vermutet wird, ebenso der Verdauungstrakt, zum Beispiel beim Reizdarmsyndrom.

Die atopische Dermatitis (ICD-10 L20), auch atopisches Ekzem oder Neurodermitis genannt, ist eine weit verbreitete Art der Dermatitis. Sie ist eine chronische Hautkrankheit, die mit entzündlichen Prozessen in Zusammenhang steht (Leung et al., JCI 2004, PMID: 14991059). Die Symptome umfassen Ekzeme auf der Haut und starken Juckreiz. Die Lebenszeitprävalenz der atopischen Dermatitis ist 10-20% in Kindern und 1-3% in Erwachsenen, wobei sich die Prävalenz in Industriestaaten in den letzten Jahrzehnten fast verdreifacht hat.

Die Krankheit gilt zwar als unheilbar, jedoch als behandelbar. Übliche Behandlungen umfassen die Behandlung der charakteristischen Hauttrockenheit, z.B. durch Feuchtigkeitscremes ("moisturizer" und/oder "emollients"), und die Gabe von entzündungshemmenden Wirkstoffen, meist in Form von Salben oder ähnlichem, direkt auf das Ekzem, die Läsion oder auf die empfindlichen Hautstellen, die zur Ekzembildung neigen.

Eine bewährte Wirkstoffklasse in der Behandlung von atopischer Dermatitis sind Corticosteroide zur topischen Anwendung. Allerdings ist deren langfristige Anwendung mit möglichen Nebenwirkungen verbunden und daher üblicherweise für die Behandlung von Hautstellen ohne Läsionen oder minderschweren Ausprägungsformen der Krankheit nicht angezeigt. Weitere Behandlungsmethoden sind beispielsweise im bereits zitierten Artikel von Leung et al. und in Akdis et al. (Allergy 2006, PMID: 16867052) aufgelistet.

Allein die Vielzahl der unterschiedlichen Behandlungsmethoden für atopische Dermatitis zeigt, dass für einen guten Behandlungserfolg nicht jeder Patient gleich behandelt werden kann. Für die meisten Patienten ist ein mehrgleisiger Ansatz ("multi-pronged approach") für eine wirkungsvolle Behandlung vonnöten. Außerdem könnte in minderschweren Fällen, bei der Langzeitbehandlung oder bei der bloßen Behandlung von Juckreiz (z.B. um den abträglichen "itchscratch cycle" zu vermeiden oder das allgemeine Wohlbefinden des Patienten zu verbessern) die Gabe von Medikamenten wie Corticosteroiden oder Calcineurin-Inhibitoren mit vergleichsweise zu hohem Risiko in Bezug auf mögliche Nebenwirkungen verbunden sein. Insbesondere die Unterdrückung von Juckreiz könnte ein effektives Mittel zur Verbesserung des Krankheitsbildes sein.

Eine Aufgabe der vorliegenden Erfindung ist nun, ein Präparat zur Verfügung zu stellen, dass zum einen über einen guten Wirkungsgrad gegen eines oder mehrere der eingangs erwähnten Symptome, Störungen, oder Krankheiten der Haut verfügen soll, zumindest für eine Untergruppe von Patienten. Zum anderen soll das Präparat zur topischen Anwendung geeignet sein. Auch eine Eignung für die Anwendung auf Schleimhäuten, beispielsweise gegen entzündlichen Störungen der Schleimhäute, oder zur Einnahme bei Verdauungsstörungen wäre vorteilhaft, wie auch eine Verschönerung des Hautbildes durch die Anwendung.

Des Weiteren sollte das Präparat auf bewährten Naturprodukten basieren, was bei vielen Patienten die Compliance erhöhen kann, aufgrund der oft höheren Akzeptanz im Vergleich zu Medikamenten, die nicht aus Naturprodukten erzeugt werden.

Ferner sollte das Präparat eine oder mehrere folgender Eigenschaften aufweisen, die vorteilhaft für ein gewerbliches Produkt sind: für die möglichen Anwendungen optimierte Beschaffenheit (z.B. in Bezug auf die Viskosität und pH-Wert), längere Haltbarkeit, gegebenenfalls bei gleichbleibender Beschaffenheit, und für den Anwender attraktive Eigenschaften (bezüglich Farbe, Geruch, Geschmack, Konsistenz usw.). Insbesondere Viskosität und pH-Wert sind sehr wichtig, um eine effektive Anwendung auf der Haut sicherzustellen.

Daher offenbart die vorliegende Erfindung ein neues Verfahren zur Herstellung eines Präparats aus Hafer(Avena sativa)-Mehl (also einem bewährten Naturprodukt) und *Carica* papaya-Früchten, umfassend:
a) Aufschlämmen des Hafermehls in vorzugsweise kaltem Wasser;
b) Erhitzen der Aufschlämmung unter Rühren auf Kochtemperatur;
c) Kochen der Aufschlämmung, vorzugsweise unter Rühren, für mindestens 30 Minuten, bevorzugt für mindestens 45 Minuten, mehr bevorzugt für mindestens 1 Stunde, noch mehr bevorzugt für mindestens 2 Stunden, insbesondere für mindestens 3 Stunden;
d) Zugeben eines Präparats aus *Carica* papaya-Früchten (Bestandteil A), zur, vorzugsweise kochenden, Aufschlämmung (Bestandteil B);
wobei das Hafermehl einen durchschnittlichen Partikeldurchmesser von weniger als 2000µm, bevorzugt weniger als 1500µm, mehr bevorzugt weniger als 1000µm, noch mehr bevorzugt weniger als 500µm und insbesondere weniger als 350µm aufweist.

Weiters betrifft die vorliegende Erfindung ein Präparat erhältlich nach dem erfindungsgemäßen Verfahren. Im übrigen betrifft die vorliegende Erfindung die bereits beschriebenen Präparate zur Anwendung in der Behandlung von Krankheiten oder Störungen. Ferner betrifft die vorliegende Erfindung kosmetische nicht-therapeutische Verfahren, umfassend die Anwendung der erfindungsgemäßen Präparate.

Hafermehl wird durch Mahlen von Korn des Saat-Hafers (*Avena sativa*), einer Kulturpflanze, gewonnen. Hafermehl enthält Nährstoffe, wie auch Mineralstoffe und Vitamine, und Ballaststoffe, einschließlich Beta-Glucan, weiters Phytosterine, Alkaloide, Avenanthramide, Kieselsäure und Linolsäure. Bevorzugt ist das in der vorliegenden Erfindung verwendete Hafermehl ein Vollkorn-Hafermehl.

Kolloidales Hafermehl lässt sich aus fein gemahlenem Hafermehl und Wasser herstellen und ist Bestandteil von einigen Körperpflegeprodukten wie beispielsweise Shampoos, Feuchtigkeitscremes und Rasierschaum, zum Schutz vor Hautirritationen und gegen Trockenheit der Haut (Criquet et al., Clinical, Cosmetic and Investigational Dermatalogy 2012, PMID: 23204849). Die positiven Effekte auf Hautirritationen scheinen durch die im Hafermehl enthaltenen Avenanthramide vermittelt zu werden, die Entzündungsreaktionen verhindern. Ein weiterer Vorteil von Hafermehl ist, dass es nicht oder nur äußerst gering allergen ist.

Avenanthramide sind von besonderer Bedeutung für vorteilhaften Wirkungen des Hafers. Avenanthramide sind phenolische Substanzen, die für Hafer spezifisch sind. Sie wurden als anti-oxidativ, anti-inflammatorisch und antiatherogenetisch beschrieben (vgl. Maria Skoglund, Susanne Bryngelsson, and Lena H. Dimberg (2003). Enzymes Involved in Changes of Avenanthramide Concentrations in Steeped Oat Grains. In: Future Technologies for Food Production and Future Food Scientists. International Symposium on Future Technologies for Food Production and Future Food Scientists, Gothenburg, Sweden, (90-90). 2-4 June 2003) Eine weitere Beschreibung der Avenanthramide findet sich beispielsweise in der WO 2010/108277 A1.

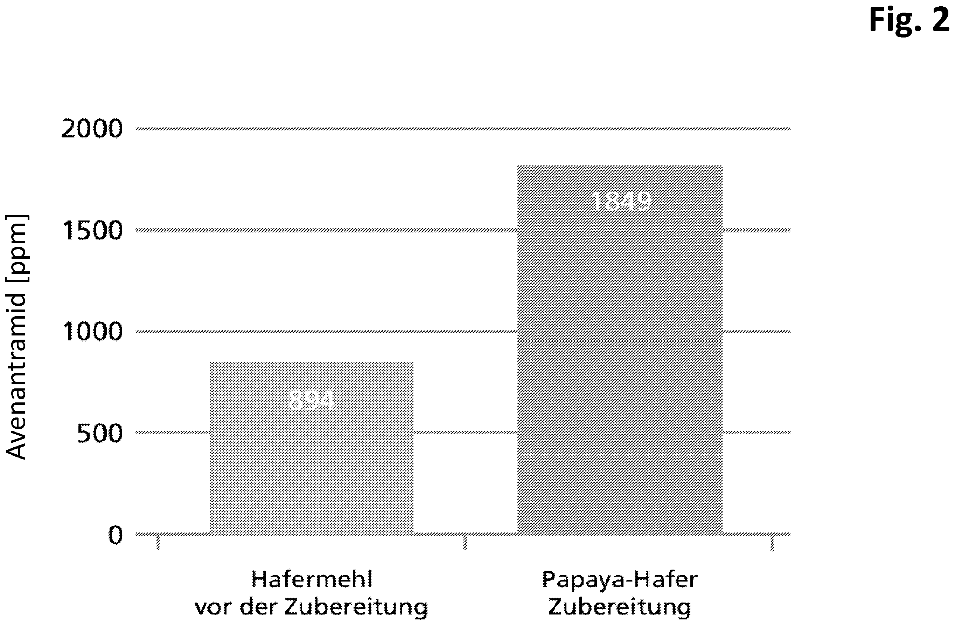

In der Nomenklatur nach Collins wird unter anderem zwischen Avenanthramid A, Avenanthramid B und Avenanthramid C unterschieden (vgl. die WO 2010/108277 A1, insbesondere Fig. 2 darin); eine weitere Nomenklatur wurde von Dimberg etabliert (darin ist z.B. Avenanthramid B als Avenanthramid 2f bezeichnet; Wise, Mitchell L. "Avenanthramides: Chemistry and Biosynthesis." Oats Nutrition and Technology (2013): 195-226). Sie alle werden aus im Hafersamen befindlichen Vorstufen durch das Enzym Hydroxycinnamoyl-CoA:hydroxyanthranilat N-Hydroxycinnamoyltransferase (HHT) synthetisiert - die Konzentration von Avenanthramid bzw. der Vorstufen davon kann, je nach Hafersorte und Wachstumsbedingungen, unterschiedlich sein. HHT ist während der Keimung aktiv. Durch Aufquellenlassen von intakten Hafersamen in Wasser wird die Avenanthramid-Konzentration erhöht. Die optimalen Bedingungen für die HHT-Enzymaktivität sind gemäß Skoglund et al., 2003, pH 7 und 30°C.

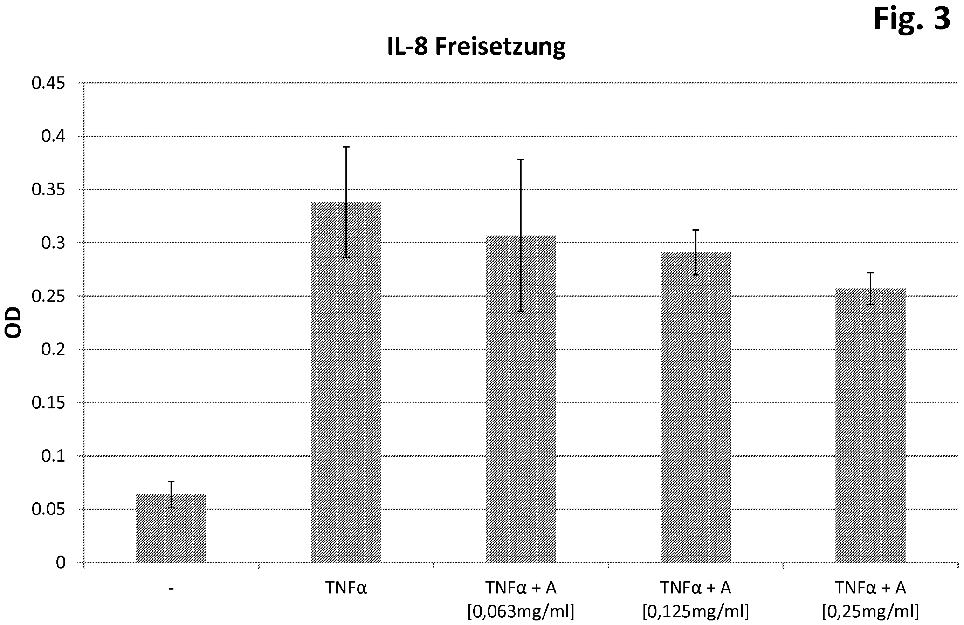

Der Stand der Technik schlägt deshalb das lange Aufquellenlassen in Wasser bei Temperaturen weit unter der erfindungsgemäß vorgesehenen Kochtemperatur (also z.B. etwa 100°C bei Normaldruck) vor, um die Avenanthramid-Konzentration in Haferendprodukt zu erhöhen. Beispielsweise wird in der WO 2010/108277 A1 ein Aufquellenlassen in Wasser bei einer Temperatur von 4 bis 40°C für 96 bis 120 Stunden (vgl. Anspruch 5 jenes Dokuments) empfohlen. Skoglund et al., 2008, (Skoglund, Maria, et al. "Avenanthramide content and related enzyme activities in oats as affected by steeping and germination." Journal of cereal science 48.2 (2008): 294-303.) zeigt ebenfalls eine Konzentrationszunahme der Avenanthramide, die erst nach 96-120 Stunden Keimung bei 16°C bzw. 20°C besonders bemerkenswert ist (Fig. 3 ebenda).

Im Zuge der vorliegenden Erfindung wurde jedoch herausgefunden, dass sich durch längeres Kochen, vorzugsweise unter Rühren, (mindestens 30 Minuten) - nach kurzem Aufschlämmen - von fein gemahlenem Hafermehl, insbesondere mit einem durchschnittlichen Partikeldurchmesser zwischen 200pm und 400pm, ebenso eine Erhöhung der Avenanthramidkonzentration erreichen lässt. Gemessen wird diese Erhöhung durch Bestimmen der Avenanthramidkonzentration (z.B. per HPLC, vgl. Beispiel 4 der vorliegenden Anmeldung und auch WO 2010/108277 A1) in der Ausgangssubstanz (Hafermehl) und dem nach dem Kochen erhaltenen Haferpräparat, jeweils normalisiert auf die jeweilige Trockenmasse, wobei die beiden Konzentrationen (z.B. jeweils angegeben in ppm) miteinander verglichen werden. Das erfindungsgemäße Verfahren ermöglicht solch eine Erhöhung der (Gesamt-)Avenanthramidkonzentration um mindestens 10%, bevorzugt um mindestens 20%, mehr bevorzugt um mindestens 50%, insbesondere um mindestens 100% (also eine Verdopplung), wobei Avenanthramid B vorzugsweise einen Anteil an besagten (Gesamt-)Avenanthramiden hat, bevorzugt von mindestens 5%, mehr bevorzugt von mindestens 10%, noch mehr bevorzugt von mindestens 20%, insbesondere von mehr als 50% oder gar mehr als 75%. Anhand von Beispiel 4 (unten) wird die erfindungsgemäße Erhöhung der Avenanthramidkonzentration gezeigt.

Zwar finden sich im Stand der Technik einige Dokumente, die das längere Kochen von Haferflocken beschreiben, keines jedoch offenbart das erfindungsgemäße längere Kochen von Hafermehl, geschweige den das erfindungsgemäße längere Kochen zum Zwecke der Erhöhung der Avenanthramid-Konzentration. Hinzu kommt, dass das englische Wort "oatmeal" mehrdeutig ist und sowohl Haferflocken (auch "rolled oats") als auch Hafermehl (auch "oat flour") bedeuten kann. So ist jede Offenbarung, welche das Wort "oatmeal" ohne genauere Spezifizierung verwendet (z.B. durch Angabe der durchschnittlichen Partikelgröße), nicht als eindeutig zu betrachten im Bezug darauf, ob damit Haferflocken oder Hafermehl gemeint ist.

So offenbart die US 1,414,576 ein Zubereitungsverfahren für Haferprodukte. Als Kochzeit vorgesehen wird eine Dauer von vier bis sechs Stunden für "dry oatmeal or rolled oats, that is oats from which the hulls have been removed". Der Ausdruck "oatmeal" wird nicht näher spezifiziert in Bezug auf die Partikelgröße bzw. den Mahlgrad. Zudem wird im Verfahren von US 1,414,576 das "oatmeal" nicht erst in (vorzugsweise kaltem) Wasser aufgeschlämmt, wie dies im erfindungsgemäßen Verfahren der Fall ist. Avenanthramide werden im Dokument nicht erwähnt.

Die US 4,765,981 beschreibt einen Extrakt aus "oatmeal". Der Ausdruck "oatmeal" wird nicht näher spezifiziert in Bezug auf die Partikelgröße bzw. den Mahlgrad, die Kochzeit beträgt mindestens 1 Stunde, vorzugsweise 3 Stunden, vor der Extraktion durch Filtrierung. Avenanthramide werden im Dokument nicht erwähnt.

Die US 2013/0183405 A1 beschreibt die Herstellung von "löslichem" Hafermehl ("soluble oat flour"; werden zur Herstellung von Hafergetränken ("oat beverages") verwendet) mit - gegenüber anderen "löslichen" Hafermehlen - erhöhter Avenanthramid-Konzentration. Lösliches Hafermehl wird durch enzymatischen Aufschluss von Hafermehl (z.B. durch alpha-Amylase) hergestellt. Dabei wird Hafermehl erwärmt, um eine Stärke-Gelierung zu erzielen, worauf das gelierende Mehl enzymatisch behandelt wird (insbesondere alpha-Amylase). Dann wird das enzymatisch behandelte lösliche Hafermehl unter Erhitzung extrudiert. Dabei wird aber das lösliche Hafermehl allerdings weder in Wasser aufgeschlämmt noch einem Kochschritt, insbesondere einem längeren Kochschritt, unterzogen. Außerdem wird bei dem in der US 2013/0183405 A1 beschriebenen löslichen Hafermehl nur eine Maximalzunahme bei Avenanthramid 2f (entspricht Avenanthramid B) von 18.31% erreicht (vgl. Tabelle in Absatz [0064] im Dokument). Diese moderate Erhöhung des Aventhramit-Gehaltes wird gemäß der in der Tabelle in Absatz [0064] der US 2013/0183405 A1 vermittelten technischen Lehre vor allem durch eine Balance der eingesetzten Enzymmenge sowie der Optimierung des Feuchtigkeitsgehaltes des löslichen Hafermehls und des Extrusionsschrittes erzielt.

Mit dem erfindungsgemäßen Verfahren werden hingegen Zunahmen von über 100% erreicht (vgl. Beispiel 4). Zudem erfordert das im Dokument beschriebene Verfahren einen Extruder, was erhöhten technischen Aufwand bedeutet. Schließlich besteht die Aufgabe der vorliegenden Erfindung auch nicht darin, ein lösliches Hafermehl zur Verfügung zu stellen (worin Avenanthramide im Zuge des Löslichkeitsprozesses angereichert werden sollen), sondern darin, ein verbessertes Hafermehlprodukt bereitzustellen. Demgemäß ist gemäß dem Verfahren der vorliegenden Erfindung eine enzymatische Behandlung, insbesondere ein enzymatischer Stärkeabbau (z.B. durch alpha-Amylasen) nicht erforderlich bzw. vorgesehen, ebensowenig wie eine Extrusion, insbesondere eine Hitzeextrusion. Im erfindungsgemäßen Verfahren wird daher vorzugsweise eine Behandlung mit Enzymen, d.h. eine Zugabe von Enzympräparationen, beispielsweise zum Abbau von Stärkemolekülen im Mehl, und/oder ein Extrusionsschritt, insbesondere eine Hitzeextrusion, vermieden bzw. wäre sogar widersinnig im Hinblick auf die erfindungsgemäß bevorzugt herzustellenden Produkte.

Die US 2005/0042243 A1 beschreibt ein Verfahren zur Herstellung eines Extrakts aus Haferprodukt ("oatmeal"). Der Ausdruck "oatmeal" wird nicht näher spezifiziert in Bezug auf die Partikelgröße bzw. den Mahlgrad. Es handelt sich beim beschriebenen Verfahren um eine Lösungsmittelextraktion ohne Kochen. Die Avenanthramidkonzentration im Endprodukt wurde zwar gemessen, allerdings wurde keine Avenanthramid-Konzentrationserhöhung im Vergleich zum Ausgangsprodukt beschrieben, anders als im erfindungsgemäßen Verfahren.

Die bereits erwähnte WO 2010/108277 A1 schlägt das Aufquellenlassen bei 4°C-40°C, vorzugsweise für 96-120 Stunden vor, um die Avenanthramid-Konzentration zu erhöhen.

Die CN 103461439 A, die US 2009/311376 A1 und die WO 2006/069390 A2 offenbaren jeweils Zubereitungen von Hafermehl, nehmen aber die vorliegende Erfindung nicht vorweg und führen auch nicht zu ihr hin. Insbesondere beschreibt keines der Dokumente das Zugeben eines Präparats aus *Carica papaya-*Früchten (d.h. Bestandteil A) zu einer (Hafermehl beinhaltenden) Aufschlämmung (d.h. Bestandteil B).

Die erfindungsgemäße Verwendung von Hafermehl (statt Haferflocken) erlaubt einen besseren Aufschluss der wertvollen Inhaltsstoffe des Hafers und ein homogeneres Endprodukt. Ein homogenes Erscheinungsbild ist sowohl für die Handhabung (z.B. Abfüllung) als auch, insbesondere bei Anwendung auf die Haut, für den Konsumenten sehr wichtig.

Daneben hat das erfindungsgemäße Verfahren gegenüber dem Stand der Technik den Vorteil, dass es kürzer dauert und eine gleichzeitige hygienische Behandlung durch das Halten auf Kochtemperatur erlaubt.

Hafermehl ist nicht nur zur Anwendung auf der Haut geeignet: Haferprodukte wie Hafermehl oder auch Haferflocken sind bekömmlich und können einen wichtigen Bestandteil gesunder Ernährung bilden. Deren hoher Ballaststoffanteil kann das Risiko für Bluthochdruck, Fettstoffwechselstörungen und Herzerkrankungen verringern. Im allgemeinem haben Personen mit höherem Ballaststoffanteil in der Ernährung weniger chronische Krankheiten (Slavin, Journal of the American Dietetic Association 2008, PMID: 18953766; abstract). Hafermehl hat anti-oxidative und anti-inflammatorische Eigenschaften (Kurtz and Wallo, J Drugs Dermatol 2007, PMID: 17373175; abstract), was sich beruhigend auf Verdauungstörungen auswirken kann.

Weiters kann eine Erhöhung des Anteils von Haferprodukten in der täglichen Ernährung die Insulinresistenz bei Typ-2-Diabetes verringern und dadurch zu einer Verbesserung des Krankheitsbildes führen. So untersucht beispielsweise Youn et al (Youn, Moonyeon, A. Saari Csallany, and Daniel D. Gallaher. "Whole grain consumption has a modest effect on the development of diabetes in the Goto-Kakisaki rat." British Journal of Nutrition 107.02 (2012): 192-201.) den Effekt von Haferprodukten auf Typ-2-Diabetes im Tiermodell.

Im Zuge der vorliegenden Erfindung wurden verschiedene Inhaltsstoffe, Formulierungen bzw. Herstellungsverfahren insbesondere hinsichtlich ihrer Wirksamkeit bei Hautstörungen oder Erkrankungen getestet. Letztlich hat sich das erfindungsgemäße Präparat als besonders geeignet zur Behandlung von Hautbeschwerden herausgestellt (insbesondere mit einer Papaya-Komponente wie weiter unten beschrieben, siehe auch Beispiel 2).

Überraschenderweise hat sich im Zuge der vorliegenden Erfindung herausgestellt, dass durch die Zugabe eines Präparats aus *Carica* papaya-Früchten (Bestandteil A), vorzugsweise ein Mus-Präparat aus *Carica* papaya-Früchten, zur, vorzugsweise kochenden, Aufschlämmung die Avenanthramid-Konzentration im Bezug auf die Trockenmasse des Hafermehls erhöhen lässt (siehe Beispiel 4). So wurde bei einer Zugabe von Bestandteil A bis zu einem Endanteil von 20% der Gesamttrockenmasse eine Erhöhung der Avenanthramid-Konzentration um 10% in Bezug auf die Hafermehl-Trockenmasse erhöht. Dies könnte sich dadurch erklären lassen, dass Bestandteile der Papaya das Enzym HHT aktivieren oder stabilisieren, was zu einer erhöhten Umsetzung zu Avenanthramiden führt.

Carica papaya (Melonenbaum) gehört zur Familie Caricaceae der Violales und produziert große, saftige und wohlschmeckende Früchte (Papayas).

Die Papaya stammt aus tropischen Regionen, wo sie auch kultiviert wurde. Großangelegte Plantagen findet man in Ceylon, Pakistan, Indien, Australien, Ostafrika und Brasilien. In Mexiko und Zentralamerika findet man ebenso viele Plantagen, die jedoch wesentlich kleiner sind. Der Baum wird bis zu sechs Meter hoch, die Früchte können ein Gewicht von bis zu 7 kg erreichen.

In traditionellen medizinischen Kulturen wird Papaya (Schale, Fruchtfleisch, Samen; selten auch Blätter und Latex) vor allem bei Asthma, Parasitosen, Wundheilungsstörungen sowie gastrointestinalen Problemen wie Diarrhoe oder Obstipation eingesetzt. Die Inhaltsstoffe stimulieren und regulieren die Verdauungstätigkeit, mildern eine Übersäuerung des Magens, vermindern eine zu starke Gasbildung und unterstützen die Aufspaltung von Proteinen.

Über mögliche Heilwirkungen wurde erstmals schriftlich vom Spanier Oviedo berichtet (1526). Dr. Mario Rojas Alba, Präsident des mexikanischen Institutes für Traditionelle Medizin beschäftigt sich seit 1996 intensiv mit der heilsamen Wirkung dieser Frucht.

Bisher wurden sechs verschiedene Enzyme isoliert: Papain, Chymopapain A und B, Lysozyme, Lipase, Glutamin-Cyclopherase, Kailose.

Weiters ist die Papaya sehr reich an: Pektin, Vitamin A, B und C, Essentiellen Fettsäuren, Bioflavonoiden, Kalium, Kalzium, Magnesium, Phosphotiden, Peptiden, Aminosäuren (z.B. Arginin) .

Das Glykosid Carpain soll einen herzmuskelstärkenden Effekt aufweisen.

Neben der Verwendung als Lebensmittel werden die Früchte von *Carica papaya* zur Herstellung des proteolytischen Enzyms Papain verwendet.

Papain wurde zur Verhinderung von Brandwundeninfektionen, zur Defibrination von Wunden, zur Behandlung von Insektenstichen, zur Behandlung von Ödemen, entzündlichen Prozessen und zur Beschleunigung der Wundheilung sowie - in geringen Dosierungen - bei Magenverstimmungen verwendet. Papayas werden weiters als laxativ und erfrischend beschrieben.

Papain (Papaya peptidase I, EC 3.4.22.2) wird dabei aus dem Milchsaft (Latex) unreifer Papayas gewonnen, wobei dieser Milchsaft eingetrocknet und pulverisiert wird.

Sowohl Papaya-Präparate als auch Hafermehl-Präparate eignen sich grundsätzlich durch ihre zahlreichen positiven Eigenschaften zur äußeren und inneren Anwendung bei Mensch und Tier.

Wegen all der genannten Vorteile, insbesondere der vorteilhaften Wirkung auf die Avenanthramid-Konzentration, umfasst das erfindungsgemäße Verfahren den oben genannten Schritt d). Vorzugsweise ist das Präparat aus *Carica papaya-*Früchten (Bestandteil A) ein Mus-Präparat aus *Carica papaya-*Früchten.

Vorzugsweise ist dieser Verfahrensschritt dadurch gekennzeichnet, dass das Zugeben von Bestandteil A unter Rühren und/oder bis zu einer Gesamtkonzentration von Bestandteil A von 5%-50% (v/v), vorzugsweise 10%-40% (v/v), insbesondere 15%-25% (v/v) erfolgt.

Eine kommerziell erfolgreiche Verwendung eines Mus-Präparats aus Papaya-Früchten ist in der WO 2004/047850 A1 offenbart. Es handelt sich dabei um ein Präparat, das durch das spezielle Herstellungsverfahren einen gegenüber herkömmlichen Papaya-Präparaten Präparaten höheren Wirkungsgrad aufweisen kann (hierin auch "das spezielle Mus-Präparat", "das spezielle Papaya-Mus-Präparat", "das spezielle Mus-Präparat aus Carica papaya-Früchten" oder "das spezielle Papaya-Präparat" genannt). Es ist unter anderem hilfreich bei Verdauungsstörungen.

Bislang war eine Verwendung des speziellen Papaya-Mus-Präparats auf der Haut nicht bekannt, im Zuge der vorliegenden Erfindung wurde die Eignung des erfindungsgemäßen Präparats (umfassend das spezielle Papaya-Mus-Präparat) zur Anwendung bei entzündlichen Störungen der Haut getestet. Dabei hat sich das erfindungsgemäße Präparat (umfassend das spezielle Papaya-Mus-Präparat), als besonders geeignet herausgestellt, vor allem wegen der anti-inflammatorischen und beruhigenden Wirkungen des Papaya-Präparats, welche durch die Hafermehl-Komponente (z.B. die darin enthaltenen Avenanthramide) ergänzt wird, und mit der sich auch die Viskosität des Präparats gut steuern (und dadurch verbessern) lässt, was für die Anwendung auf der Haut sehr wichtig ist. Auch eine vorteilhafte Wirkung auf die Avenanthramid-Konzentration lässt sich beobachten.

Deshalb ist eine bevorzugte Ausführungsform des erfindungsgemäßen Verfahrens dadurch gekennzeichnet, dass Bestandteil A ein Mus-Präparat aus *Carica* papaya-Früchten ("das spezielle Papaya-Mus-Präparat") ist, erhältlich nach einem Verfahren, gekennzeichnet durch die folgenden Schritte:
- Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
- Abkühlen der gekochten Früchte oder zerkleinerten Früchte über einen Zeitraum von zumindest 30 min unter Sauerstoff-haltiger Atmosphäre, vorzugsweise unter Luftzufuhr und bei Raumtemperatur,
- gegebenenfalls Zerkleinern, Mischen und Passieren der abgekühlten Früchte oder zerkleinerten Früchte bis zum Erhalt eines homogenen Muses.

Vor allem die Kombination der wirksamen Inhaltsstoffe von speziellem Papaya-Mus-Präparat und Hafermehl ist für die therapeutischen Eigenschaften der vorliegende Erfindung von herausragender Bedeutung. Das spezielle Herstellungsverfahren vervielfacht daher die positive Wirkung der Papayafrucht auf Haut und/oder Schleimhaut, wie es auch die auch die vom Hafer bekannte antibakterielle, fungizide und entzündungshemmende Wirkung verbessern kann. Das erfindungsgemäße Präparat kann zusammenfassend unter anderem folgende Eigenschaften und Effekte aufweisen: UV-absorbierend, reinigender Effekt (durch Papaya-Mus-Präparat), immunregulierend, Ceramid-Synthesestimulierend, feuchtigkeitsspendend (durch Hafermehl), entzündungshemmend, schmerzlindernd, anti-oxidativ, wundheilungsfördernd, Juckreiz-reduzierend und Irritationsreduzierend (durch Zusammenwirken beider Bestandteile). Das erfindungsgemäße Präparat kann während der Schwangerschaft und Stillzeit angewendet werden und ist auch für Babys und Kleinkinder geeignet.

Die CN 102406042 A offenbart eine Eiscreme zur Gewichtsreduktion mit Papaya und Hafer (also Haferflocken oder Haferkörner, nicht jedoch Hafermehl!). Daher nimmt das Dokument die vorliegende Erfindung nicht vorweg und führt auch nicht zu ihr hin.

Die CN 103519078 A offenbart ein Gebäck aus Papaya-Saft und Hafermehl. Insbesondere die Kochzeit einer Hafer-beinhaltenden Aufschlämmung wird jedoch nicht offenbart. Außerdem ist zwischen Papaya-Saft und Papaya-Mus zu unterscheiden, wie weiter unten in der vorliegenden Beschreibung erläutert wird. Daher nimmt das Dokument die vorliegende Erfindung nicht vorweg und führt auch nicht zu ihr hin.

Die CN 102524640 A offenbart ein Pulver zur Darmreinigung, das unter anderem Hafermehl und Papaya beinhaltet. Insbesondere ein Kochschritt wird jedoch nicht offenbart. Daher nimmt das Dokument die vorliegende Erfindung nicht vorweg und führt auch nicht zu ihr hin.

Es mögen zwar diverse Hausmittel oder Ähnliches bekannt sein, in denen Papaya-Mus und Haferflocken (vielleicht auch Hafermehl) beispielsweise zur Anwendung auf der Haut, vorwiegend direkt vor der Anwendung, kombiniert werden, doch die vorliegende Erfindung unterscheidet sich davon in einem oder mehreren der folgenden Gesichtspunkte:
- In der vorliegenden Erfindung wird die Aktivität der Inhaltsstoffe, insbesondere des in Bestandteil A enthaltenen Papains, besonders gut erhalten.
- In der vorliegenden Erfindung kann der pH durch Zitronensaftkonzentrat oder ein anderes genießbares Säuerungsmittel auf ein besonders verträgliches Niveau (beispielsweise für die Haut oder Schleimhaut) gesenkt werden.
- In der vorliegenden Erfindung wird Hafermehl (statt Haferflocken) verwendet, was zum einen zu besserem Wirkstoffaufschluss und zum anderen zu gleichmäßigerer Konsistenz führt. Außerdem enthält Hafermehl mehr Folsäure im Vergleich zu Haferflocken, laut: http://www.ernaehrung.de/lebensmittel/de/C233000/HaferMehl.php und http://www.ernaehrung.de/lebensmittel/de/C133000/HaferFlocken.php
   Folsäure ist gegen chronisch entzündliche Hautkrankheiten wie beispielsweise Psoriasis hilfreich (Gisondi et al, J Dermatolog. Treat. 2007, PMID: 17538801, abstract).
- In der vorliegenden Erfindung wird das aufgeschlämmte Hafermehl vorzugsweise unter ständigem Rühren gekocht, was die Eignung der Erfindung für die großtechnische Anwendung erhöht, weil unter anderem die Viskosität besonders gut kontrolliert werden kann und das Verfahren der Erfindung durch das Kochen auch hygienische Vorteile, beispielsweise in Bezug auf Haltbarkeit, bringt. Hausmittel dagegen sind üblicherweise so konzipiert, dass sie direkt vor der Anwendung zubereitet werden. Daher eignen sie sich nicht für großtechnische Herstellung eines gewerblichen Produkts.
- Oftmals wird statt Papaya-Mus im Stand der Technik nur Papaya-Saft (d.h. ohne Pulpe) verwendet. Dadurch gehen viele wertvolle Inhaltsstoffe verloren. Ausführungsformen des erfindungsgemäßen Präparats sehen hingegen die Zugabe von Papaya-Mus (d.h. mit Pulpe) vor.

Die mit der vorliegenden Erfindung gezeigten Effekte des erfindungsgemäßen Präparats zur Erzielung der Hautverbesserungen sind auf seine beruhigende, entzündungshemmende, schmerzlindernde, feuchtigkeitsspendende Wirkung, sowie seine positiven Effekte auf die Wundheilung zurück zu führen.

Das erfindungsgemäße Präparat zeichnet sich durch kontrollierte und gleichmäßige Viskosität und durch hygienische Vorteile (u.a. durch das Kochen), beispielsweise in Bezug auf Haltbarkeit, aus.

Beispielsweise kann das Erhitzen und Kochen in einem doppelwandigen Kessel mit Umlauferhitzer erfolgen, zur besseren Temperaturregulierung. Beim Abkühlprozess ist Sauerstoffzufuhr wichtig, vorzugsweise wird unter Normaldruck bzw. Luft, insbesondere unter Luftzufuhr, gearbeitet.

Bestandteil A an sich ist bereits in der WO 2004/047850 A1 offenbart, wie auch eine detaillierte Offenbarung des Herstellungsverfahrens, der Produkteigenschaften und der Anwendung. Ebenso lehrt die WO 2004/047850 A1 ein konkretes Ausführungsbeispiel für ein Herstellungsverfahren von Bestandteil A.

In einer weiteren bevorzugten Ausführungsform kann das erfindungsgemäße Verfahren ferner einen oder mehrere der folgenden Schritte umfassen:
e) Zugeben von Wasser (erlaubt das Einstellen der Viskosität zur Verbesserung der Präparats), vorzugsweise nach Schritt c) oder d), vorzugsweise bis zu einem Gesamtwassergehalt (v/v) des Präparats zwischen 75% und 95%, vorzugsweise zwischen 80% und 93%, insbesondere zwischen 85% und 90%;
f) Halten des Gemischs auf 70°C-90°C, insbesondere auf 75°C-85°C für mindestens 10 Minuten (erlaubt unter anderem eine besonders gute Durchmischung unter hygienischen Bedingungen), vorzugsweise nach Schritten c) oder d) oder e);
g) Zugeben von Bindemittel(n) (erlaubt das Einstellen der Viskosität), Aromastoff(en) (kann das Produkt annehmbarer für den Patienten oder Verbraucher machen) und/oder Konservierungsmittel(n), vorzugsweise nach Schritten c) oder d) oder e) oder f);
h) Einstellen des pH-Werts durch Zugeben von Zitronensaftkonzentrat, Zitronensäure und/oder einem anderen genießbaren Säuerungsmittel auf 4 bis 6, vorzugsweise 4,5 bis 5,5 (unter anderem besonders wichtig für die Anwendung auf der Haut, da ein leicht saurer pH-Wert dem physiologischen pH-Wert der Haut entspricht und möglicherweise wichtig für eine gute Haltbarkeit des Produkts), vorzugsweise nach Schritten c) oder d) oder e) oder f) oder g).

Die jeweiligen Kochschritte für Papaya- bzw. Haferkomponente nach dem erfindungsgemäßen Verfahren müssen für mindestens 30 Minuten durchgeführt werden, weil ansonsten nur ein unzureichender Aufschluss erzielt werden könnte. Bevorzugt wird der jeweilige Kochschritt jeweils unabhängig voneinander aber für mindestens eine Stunde, noch bevorzugter für mindestens zwei Stunden, insbesondere für mindestens drei Stunden durchgeführt. Insbesondere der Hafer-Kochschritt wird für mindestens 30 Minuten, bevorzugt für mindestens 45 Minuten, mehr bevorzugt für mindestens 1 Stunde, noch mehr bevorzugt für mindestens 2 Stunden, insbesondere für mindestens 3 Stunden durchgeführt; u.a. für die Erhöhung der Avenanthramid-Konzentration. In weiteren Ausführungsformen wird der Hafer-Kochschritt für mindestens 4 Stunden, bevorzugt für mindestens 5 Stunden, mehr bevorzugt für mindestens 6 Stunden, noch mehr bevorzugt für mindestens 8 Stunden, insbesondere für mindestens 10 Stunden durchgeführt. Aus verfahrenstechnischen Gründen empfiehlt es sich, den jeweiligen Kochschritt für Papaya- bzw. Haferkomponente nicht unbedingt länger als 24 Stunden, vorzugsweise nicht länger als 12 Stunden, insbesondere nicht länger als 8 Stunden durchzuführen und dann - im Falle der Papaya-Komponente - vorzugsweise auf Raumtemperatur auskühlen zu lassen. Der Abkühlungsschritt wird vorzugsweise bei Raumtemperatur (20°C) und Normaldruck in normaler Atmosphäre so lange durchgeführt, bis das Präparat auf zumindest 40°C, insbesondere zumindest 30°C ausgekühlt worden ist.

Gemäß einer bevorzugten Ausführungsform betrifft die vorliegende Erfindung in einem weiteren Aspekt ein Präparat, erhältlich nach dem erfindungsgemäßen Verfahren, wobei das Hafermehl vorzugsweise als wässriges Kolloid eingesetzt wird. Insbesondere bei guter Durchmischung (z.B. besonders langer Rührzeit) liegt der wesentliche Großteil des Hafermehls als Kolloid vor. Wenn das Hafermehl fein gemahlenes Hafermehl ist (gegebenfalls mit einem durchschnittlichen Partikeldurchmesser von weniger als 2000µm, vorzugsweise weniger als 1500µm, insbesondere weniger als 1000µm oder gar 500pm), unterstützt das die Kolloidbildung in besonderem Maße und führt zu einer besonders gleichmäßigen Beschaffenheit, einschließlich einer gleichmäßigen Viskosität. Bei nicht ganz runden Partikeln ist mit "Durchmesser" die Länge der längsten Ausdehnung der Partikel zu verstehen. Vorzugsweise enthält das erfindungsgemäß zum Einsatz kommende Hafermehl im Wesentlichen (also z.B. unter 0,1%) keine Partikel, die größer sind als 5000pm, noch bevorzugter im Wesentlichen keine Partikel, die größer als 2000µm, insbesondere im Wesentlichen keine Partikel, die größer sind als 500 µm.

Zwar ist das erfindungsgemäße Präparat mit dem speziellen Papaya-Mus aus den hierin dargelegten Gründen exzellent geeignet zur Erfüllung der erfindungsgemäßen Aufgaben, doch eine andere Ausführungsform des erfindungsgemäßen Präparats, vorzugsweise in dem das Papaya-Präparat auf andere als die bereits erwähnten Weisen zugegeben bzw. zubereitet wurde, ist ebenfalls zur Erfüllung der erfindungsgemäßen Aufgaben geeignet.

In einer weiteren bevorzugten Ausführungsform der vorliegenden Erfindung ist das Präparat durch das Kochen hygienisch. "hygienisch" bedeutet vorzugsweise, dass das Präparat die europäischen oder österreichischen Toleranz- bzw. Grenzwerte für Keimbelastung in Lebensmitteln oder Nahrungsergänzungsmitteln nicht überschreitet. Insbesondere bedeutet hygienisch, dass weniger als 1000000 Koloniebildende Einheiten (KBE) mesophiler, aerober Keime pro g Präparat vorkommen, bevorzugt weniger als 100000 KBE pro g, mehr bevorzugt weniger als 10000 KBE pro g, noch mehr bevorzugt weniger als 1000 KBE pro g.

In einer weiteren bevorzugten Ausführungsform der vorliegenden Erfindung ist das Präparat dadurch gekennzeichnet, dass die Konzentration der Avenanthramide in Bezug auf die Gesamttrockenmasse des Präparats mindestens 500ppm, bevorzugt mindestens 1000ppm, mehr bevorzugt mindestens 1250ppm, noch mehr bevorzugt mindestens 1500ppm, insbesondere mindestens 1750ppm beträgt;
vorzugsweise wobei Avenanthramid B einen Anteil an besagten Avenanthramiden hat, bevorzugt von mindestens 5%, mehr bevorzugt von mindestens 10%, noch mehr bevorzugt von mindestens 20%, insbesondere von mehr als 50% oder gar mehr als 75%.

In einer weiteren bevorzugten Ausführungsform umfasst das erfindungsgemäße Präparat ferner Zitronensaftkonzentrat, Konservierungsmittel, Speisestärke, Aromastoff(en) und/oder Wasser, gegebenenfalls ohne weitere Inhaltsstoffe. Der Ausdruck "ohne weitere Inhaltsstoffe" bedeutet im Kontext der vorliegenden Anmeldung, dass diese weiteren Inhaltsstoffe, wenn überhaupt, nur in zu vernachlässigenden Spuren enthalten sind, beispielsweise in einer Konzentration von weniger als 1 oder 10 oder 100 ppm (parts per million).

Die eben genannten Inhaltsstoffe können die Eignung des Präparats zum gewerblichen Produkt verbessern, beispielsweise durch für die möglichen Anwendungen optimierte Beschaffenheit (z.B. in Bezug auf die Viskosität und pH-Wert), längere Haltbarkeit, gegebenenfalls bei gleichbleibender Beschaffenheit, und für den Anwender attraktive Eigenschaften (bezüglich Farbe, Geruch, Geschmack, Konsistenz usw.).

In einer weiteren bevorzugten Ausführungsform ist die Gesamtkonzentration (v/v) von Speisestärke und Konservierungsmittel im erfindungsgemäßen Präparat unabhängig voneinander jeweils zwischen 0% und 2%, insbesondere zwischen 0.5% und 1.5%. Vorzugsweise wird das erfindungsgemäße Präparat nach im Stand der Technik bekannten Verfahren pasteurisiert. Dies führt unter anderem zur Verbesserung von Viskosität und Haltbarkeit.

In einer weiteren bevorzugten Ausführungsform ist das erfindungsgemäße Präparat dadurch gekennzeichnet, dass der Wassergehalt (v/v) des Präparats zwischen 75% und 95%, vorzugsweise zwischen 80% und 93%, insbesondere zwischen 85% und 90% liegt. Dies führt zur Verbesserung von Viskosität und Handhabung des Präparats.

In einer weiteren bevorzugten Ausführungsform ist das erfindungsgemäße Präparat dadurch gekennzeichnet, dass der pH-Wert des Präparats zwischen 4 und 6, insbesondere zwischen 4,5 und 5,5, liegt. Dies dienst zur Verbesserung der Verträglichkeit des Präparats auf der Haut, Schleimhaut, bei der Einnahme, usw.

In einer weiteren bevorzugten Ausführungsform ist das erfindungsgemäße Präparat dadurch gekennzeichnet, dass die Viskosität des Präparats zwischen 3000 und 10000 cP
(Centipoise), insbesondere zwischen 4000 und 8000 cP, liegt. Dies führt zur Verbesserung der Handhabung des Präparats.

In einer weiteren bevorzugten Ausführungsform ist das erfindungsgemäße Präparat dadurch gekennzeichnet, dass beim Mus-Präparat (insbesondere beim speziellen Papaya-Mus-Präparat) aus *Carica* papaya-Früchten:
- das Kochen mindestens 2h durchgeführt wird; und/oder
- das Abkühlen mindestens 5h durchgeführt wird; und/oder
- die Früchte vor dem Kochen geschält und entkernt werden; und/oder
- die Früchte halbreif bis reif sind;
und/oder dass das Mus-Präparat aus *Carica* papaya-Früchten pasteurisiert ist.

Dies führt zur weiteren Erhöhung der Aktivität der Inhaltsstoffe (einschließlich der Papain-Aktivität) im Mus-Präparat aus *Carica* papaya-Früchten. Die besten Resultate können mit *Carica* papaya-Früchten erzielt werden, die halbreif bis reif sind. Der Reifezustand der Papayas lässt sich anhand der Farbe der Schale definieren: Unreife Früchte haben eine 100% grüne Schale, halbreife sind 50-75% gelb, reife Früchte sind 80-100% gelb. Um ein Endprodukt zu erhalten, das in seiner Konsistenz und Form noch appetitlicher aussieht und noch besser eingenommen werden kann, können die Früchte vor dem Kochprozess geschält und entkernt werden. Bei dieser Gelegenheit können die Früchte auch gleich vor dem Kochprozess zerkleinert werden, z.B. durch Siebung. Ebenfalls kann eine anschließende Pasteurisierung des erhaltenen Muses bei üblichen lebensmitteltechnologischen Bedingungen bevorzugt sein.

Ein weiterer Aspekt der vorliegenden Erfindung betrifft die Verwendung des erfindungsgemäßen Präparats zur Anwendung in therapeutischen und/oder kosmetischen nicht-therapeutischen Verfahren.

Eine bevorzugten Ausführungsform dieses Aspekts betrifft das erfindungsgemäße Präparat zur Anwendung in therapeutischen Verfahren.

Erfindungsgemäß kann das Präparat zur Anwendung in der Behandlung von Reiz- und/oder Entzündungszuständen der Haut (oder Schleimhaut), ausgewählt aus

Dermatitis, insbesondere atopischer Dermatitis, Juckreiz, Rötung, trockener, rissiger, offener, nässender, verdickter, entzündeter Haut oder Schleimhaut, Sonnenbrand, Insektenstiche und Psoriasis,
und dadurch gekennzeichnet, dass die Anwendung vorzugsweise auf Haut oder Schleimhaut erfolgt, insbesondere direkt am Ort der Reiz- und/oder Entzündungszustände,
herangezogen werden.

Das erfindungsgemäße Präparat, insbesondere äußerlich angewendet gegen Juckreiz und/oder Rötung auf Gesicht und Körper, kann für trockene, gereizte, sensible, junge und reife Haut verwendet werden.

Weiters kann das erfindungsgemäße Präparat zur Anwendung in der Behandlung von Verdauungsstörungen, vorzugsweise ausgewählt aus der Gruppe bestehend aus chronischer Obstipation, Flatulenz, Gastritis, Sodbrennen und Reizdarmsyndrom, eingesetzt werden, vorzugsweise dadurch gekennzeichnet, dass das Präparat oral verabreicht wird. Wie bereits eingangs erwähnt eignen sich sowohl Papaya-Fruchtmus als auch Hafermehl hervorragend zum Verzehr.

Außerdem kann das erfindungsgemäße Präparat in einer weiteren Ausführungsform herangezogen werden zur Anwendung in der Behandlung von einem oder mehreren Krankheiten oder Zuständen, ausgewählt aus:
- geschwächtes Immunsystem (wobei das Immunsystem gestärkt wird)
- Wunden, insbesondere bei Ulcus cruris,
- Diabetes (wobei die Insulinresistenz bei Typ-2-Diabetes verringert wird und dadurch zu einer Verbesserung des Krankheitsbildes führt).

Im Rahmen derartiger Behandlungsverfahren wird das erfindungsgemäße Präparat in einer wirksamen Menge an den Patienten verabreicht, wobei der Patient vorzugsweise bereits eine diagnostizierte Erkrankung hat, die der erfindungsgemäßen Behandlung bedarf, oder der ein Risiko trägt (oder angenommen werden kann), dass er eine solche Erkrankung hat oder entwickelt.

Ein weiterer Aspekt der vorliegenden Erfindung betrifft ein kosmetisches nicht-therapeutisches Verfahren zur Anwendung des erfindungsgemäßen Präparats, vorzugsweise auf der Haut, zum Erhalt eines schöneren Hautbildes. Dadurch kann unter anderem unreine Haut behandelt werden und/oder unregelmäßige Hautpigmentierung ausgeglichen werden.

### Weitere Definitionen:

Der Begriff "Aromastoffe" umfasst alle in der Datenbank der Europäischen Union erfassten "Flavouring substances" (http://ec.europa.eu/food/food/chemicalsafety/flavouring/databa se/) und auch die in der Durchführungsverordnung (EU) Nr. 872/2012 der Kommission vom 1. Oktober 2012 zur Festlegung der Liste der Aromastoffe gemäß der Verordnung (EG) Nr. 2232/96 genannten Stoffe (http://eurlex.europa.eu/LexUriServ/LexUriServ.do?uri=OJ:L:2012:267:0001:0 161:DE:PDF).

Der Begriff "Konservierungsmittel" umfasst alle in der Europäischen Union zugelassenen Konservierungsstoffe, unter anderem Stoffe mit folgenden E-Nummern (laut der Liste der in der Europäischen Union zugelassenen Lebensmittelzusatzstoffe): 200, 202, 203, 210, 211, 212, 213, 214, 215, 218, 219, 220, 221-228, 232, 231, 232, 234, 235, 239, 242, 249, 250, 251, 252, 260-263, 270, 280-285, 290, 296, 297, 1105.

Der Begriff "genießbares Säuerungsmittel" umfasst alle in der Europäischen Union zugelassenen sauren LebensmittelZusatzstoffe, insbesondere Stoffe mit folgenden E-Nummern (laut der Liste der in der Europäischen Union zugelassenen Lebensmittelzusatzstoffe): E260, E270, E290, E296, E296, E330, E334, E338.

Der Begriff "Bindemittel" umfasst alle in der Europäischen Union für Lebensmittel zugelassenen Geliermittel, Verdickungsmittel oder Bindemittel, vorzugsweise Stoffe mit folgenden E-Nummern (laut der Liste der in der Europäischen Union zugelassenen Lebensmittelzusatzstoffe): E400-E499, insbesondere Xanthan (E415).

Der Begriff "Kochen" bedeutet vor allem das Halten des zu Kochenden auf einer Temperatur gleich oder über 100°C, insbesondere auf einer Temperatur von im Wesentlichen 100°C (bei Normaldruck, auf Meereshöhe). Für den Fachmann versteht sich jedoch, dass auch Kochtemperaturen von unter 100°C, bei Normaldruck, auf Meereshöhe, im Sinne der vorliegenden Erfindung sind, solange sie für den Fachmann mit dem Begriff "Kochen" vereinbar sind. Insbesondere sind dies Temperaturen (bei Normaldruck, auf Meereshöhe) von über 80°C, bevorzugt über 85°C, mehr bevorzugt über 90°C, noch mehr bevorzugt über 95°C. Allein schon aus hygienischen Gründen ist jedoch eine Kochtemperatur gleich oder über 100°C (bei Normaldruck, auf Meereshöhe), insbesondere von im Wesentlichen 100°C (bei Normaldruck, auf Meereshöhe), höchst bevorzugt.

Für den Fachmann ist natürlich auch evident, dass die jeweilige Kochtemperatur während des Kochens für eine gewisse Zeit (z.B. 20%, 10% oder 5% der Kochdauer) unter- oder überschritten werden darf, solange die Temperatur im Mittel über die Kochzeit gleich der vorgesehenen Kochtemperatur ist. Bevorzugt ist jedoch, dass die Temperatur beim Kochen im Wesentlichen konstant ist, insbesondere im Wesentlichen konstant gleich oder über 100°C, insbesondere konstant auf einer Temperatur von im Wesentlichen 100°C (bei Normaldruck, auf Meereshöhe) ist.

Zwar sind beispielsweise Avenanthramide (und andere Inhaltsstoffe) im Allgemeinen hitzeresistent, trotzdem kann für die Stabilität gewisser Inhaltsstoffe (z.B. Avenanthramid B) angezeigt sein, während des Kochens eine Temperatur von 150°C, besser 125°C, noch besser 100°C höchstens kurz zu überschreiten, vorzugsweise nicht länger als 20% der Kochzeit, bevorzugt nicht länger als 10% der Kochzeit, noch mehr bevorzugt nicht länger als 5% der Kochzeit, insbesondere überhaupt nicht während der Kochzeit.

Der Begriff "Wasser" wird in der vorliegenden Erfindung in dem zur Behandlung von Hafermehl bzw. zur Zubereitung von Hafermehlprodukten üblichen Sinn verstanden und soll demgemäß neben dem im pharmazeutischen Bereich üblicher Weise eingesetzten reinen Wasserarten, wie destilliertem oder entionisiertem Wasser, auch Leitungswasser oder elektrolythaltiges Wasser umfassen, sowie Wasser, das die zur Zubereitung von insbesondere diätetischen Hafermehlprodukten üblichen Zusatzstoffe enthält.

Der Begriff ppm bedeutet "parts per million". In Bezug auf Avenanthramide berechnen sich die ppm durch: Avenanthramidmasse[µg] / Gesamttrockenmasse[g] = Avenanthramidkonzentration [ppm].

Ein Präparat aus X umfasst definitionsgemäß alle Arten der Zubereitung, Herrichtung und weiterverarbeiten Formen von X.

Ein Mus aus einer Frucht beinhaltet definitionsgemäß die Pulpe (Fruchtfleisch) der Frucht, während ein Saft aus einer Frucht filtriert wird und die Pulpe bzw. wesentliche Teile davon nicht beinhaltet.

Es ist für den Fachmann evident, dass das "Rühren" im Rahmen des erfindungsgemäßen Verfahrens ganz oder teilweise durch andere Arten der mechanischen Agitation ersetzt werden kann, sofern diese im Wesentlichen als technisch äquivalent betrachtet werden können. Bevorzugt ist jedoch das "Rühren" im Rahmen des erfindungsmäßen Verfahrens tatsächlich als Rühren durchzuführen.

Die Erfindung wird anhand der nachfolgenden Ausführungsformen, auf die sie selbstverständlich nicht eingeschränkt ist, näher erläutert:
1. Verfahren zur Herstellung eines Präparats aus Hafer(Avena sativa)-Mehl und *Carica papaya-Früchten,* umfassend:
   a) Aufschlämmen des Hafermehls in vorzugsweise kaltem Wasser;
   b) Erhitzen der Aufschlämmung, vorzugsweise unter Rühren, auf Kochtemperatur;
   c) Kochen der Aufschlämmung unter Rühren für mindestens 45 Minuten, mehr bevorzugt für mindestens 1 Stunde, noch mehr bevorzugt für mindestens 2 Stunden, insbesondere für mindestens 3 Stunden;
   d) Zugeben eines Präparats aus *Carica* papaya-Früchten (Bestandteil A), vorzugsweise ein Mus-Präparat aus *Carica* papaya-Früchten, zur, vorzugsweise kochenden, Aufschlämmung (Bestandteil B),
   wobei das Hafermehl einen durchschnittlichen Partikeldurchmesser von weniger als 2000µm, bevorzugt weniger als 1500µm, mehr bevorzugt weniger als 1000µm, noch mehr bevorzugt weniger als 500µm und insbesondere weniger als 350µm aufweist.
2. Verfahren nach Ausführungsform 1, dadurch gekennzeichnet, dass das Zugeben von Bestandteil A unter Rühren und/oder bis zu einer Gesamtkonzentration von Bestandteil A von 5%-50% (v/v), vorzugsweise 10%-40% (v/v), insbesondere 15%-25% (v/v) erfolgt.
3. Verfahren nach Ausführungsform 2, wobei Bestandteil A ein Mus-Präparat aus *Carica* papaya-Früchten ist, erhältlich nach einem Verfahren, gekennzeichnet durch die folgenden Schritte:
   - Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
   - Abkühlen der gekochten Früchte oder zerkleinerten Früchte über einen Zeitraum von zumindest 30 min unter Sauerstoff-haltiger Atmosphäre, vorzugsweise unter Luftzufuhr und bei Raumtemperatur,
   - gegebenenfalls Zerkleinern, Mischen und Passieren der abgekühlten Früchte oder zerkleinerten Früchte bis zum Erhalt eines homogenen Muses.
4. Verfahren nach einem der Ausführungsformen 1 bis 3, ferner einen oder mehrere der folgenden Schritte umfassend:
   e) Zugeben von Wasser;
   f) Halten des Gemischs auf 70°C-90°C, insbesondere auf 75°C-85°C für mindestens 10 Minuten;
   g) Zugeben von Bindemittel(n), Aromastoff(en) und/oder Konservierungsmittel(n);
   h) Einstellen des pH-Werts durch Zugeben von Zitronensaftkonzentrat, Zitronensäure und/oder einem anderen genießbaren Säuerungsmittel auf 4 bis 6, vorzugsweise 4,5 bis 5,5.
5. Präparat, erhältlich nach einem Verfahren gemäß einer der Ausführungsformen 1 bis 4, vorzugsweise wobei das Präparat durch das Kochen hygienisch ist, und/oder vorzugsweise wobei das Hafermehl als wässriges Kolloid vorliegt.
6. Präparat nach Ausführungsform 5, wobei die Konzentration der Avenanthramide in Bezug auf die Gesamttrockenmasse des Präparats mindestens 500ppm, bevorzugt mindestens 1000ppm, mehr bevorzugt mindestens 1250ppm, noch mehr bevorzugt mindestens 1500ppm, insbesondere mindestens 1750ppm beträgt.
7. Präparat nach Ausführungsform 5, wobei die Konzentration von Avenanthramid B in Bezug auf die Gesamttrockenmasse des Hafermehls im Präparat mindestens 1000ppm, bevorzugt mindestens 1500ppm, mehr bevorzugt mindestens 1750ppm, noch mehr bevorzugt mindestens 2000ppm, insbesondere mindestens 2250ppm beträgt.
8. Präparat nach einer der Ausführungsformen 5 bis 7, ferner umfassend Zitronensaftkonzentrat, Konservierungsmittel, Speisestärke, Aromastoff(en) und/oder Wasser, gegebenenfalls ohne weitere Inhaltsstoffe.
9. Präparat nach einer der Ausführungsformen 5 bis 8, dadurch gekennzeichnet, dass die Gesamtkonzentration (v/v) von Speisestärke und Konservierungsmittel unabhängig voneinander jeweils zwischen 0% und 2%, insbesondere zwischen 0.5% und 1.5%, liegt und gegebenenfalls dadurch gekennzeichnet, dass es pasteurisiert ist.
10. Präparat nach einer der Ausführungsformen 5 bis 9, dadurch gekennzeichnet, dass der Wassergehalt (v/v) des Präparats zwischen 75% und 95%, vorzugsweise zwischen 80% und 93%, insbesondere zwischen 85% und 90% liegt.
11. Präparat nach einer der Ausführungsformen 5 bis 10, dadurch gekennzeichnet, dass der pH-Wert des Präparats zwischen 4 und 6, insbesondere zwischen 4,5 und 5,5, liegt.
12. Präparat nach einer der Ausführungsformen 5 bis 11, dadurch gekennzeichnet, dass die Viskosität des Präparats zwischen 3000 und 10000 cP, insbesondere zwischen 4000 und 8000 cP, liegt.
13. Präparat nach einer der Ausführungsformen 5 bis 13, beinhaltend das Mus-Präparat aus *Carica* papaya-Früchten erhältlich nach einem Verfahren, wobei das Verfahren zusätzlich dadurch gekennzeichnetist, dass:
   - das Kochen mindestens 2h durchgeführt wird; und/oder
   - das Abkühlen mindestens 5h durchgeführt wird; und/oder
   - die Früchte vor dem Kochen geschält und entkernt werden; und/oder
   - die Früchte halbreif bis reif sind;
   und/oder dass das Mus-Präparat aus *Carica* papaya-Früchten pasteurisiert ist.
14. Präparat nach einer der Ausführungsformen 5 bis 13, zur Anwendung in therapeutischen Verfahren.
15. Präparat nach Ausführungsform 14, zur Anwendung in der Behandlung von Reiz- und/oder Entzündungszuständen der Haut oder Schleimhaut, ausgewählt aus:
   Dermatitis, insbesondere atopischer Dermatitis, Juckreiz, Rötung, trockener, rissiger, offener, nässender, verdickter, entzündeter Haut oder Schleimhaut, Sonnenbrand, Insektenstiche und Psoriasis,
   dadurch gekennzeichnet, dass die Anwendung vorzugsweise auf Haut oder Schleimhaut erfolgt, insbesondere direkt am Ort der Reiz- und/oder Entzündungszustände.
16. Präparat nach Ausführungsform 14, zur Anwendung in der Behandlung von Verdauungsstörungen, vorzugsweise ausgewählt aus der Gruppe bestehend aus chronische Obstipation, Flatulenz, Gastritis und Reizdarmsyndrom, dadurch gekennzeichnet, dass das Präparat oral verabreicht wird.
17. Präparat nach Ausführungsform 14, zur Anwendung in der Behandlung von einem oder mehreren Krankheiten oder Zuständen, ausgewählt aus:
   - geschwächtes Immunsystem
   - Wunden, insbesondere bei Ulcus cruris,
   - Diabetes.
18. Kosmetisches Verfahren, umfassend die Anwendung des Präparats nach einer der Ausführungsformen 5 bis 13, vorzugsweise auf der Haut, zum Erhalt eines schöneren Hautbildes.
19. Verfahren zur Erhöhung des Gehaltes an Avenanthramiden, insbesondere Avenanthramid B, in einem Hafermehlprodukt, dadurch gekennzeichnet, dass Hafermehl mit einem durchschnittlichen Partikeldurchmesser von weniger als 2000µm, bevorzugt weniger als 1500µm, mehr bevorzugt weniger als 1000µm, noch mehr bevorzugt weniger als 500µm und insbesondere weniger als 350µm, in Wasser aufgeschlämmt und für mindestens 30 Minuten, bevorzugt für mindestens 45 Minuten, mehr bevorzugt für mindestens 1 Stunde, noch mehr bevorzugt für mindestens 2 Stunden, insbesondere für mindestens 3 Stunden; einem Kochschritt unterzogen wird.
20. Verfahren nach Ausführungsform 19, wobei das Hafermehl im Wesentlichen (also z.B. unter 0,1%) keine Partikel, die größer sind als 5000µm, noch bevorzugter im Wesentlichen keine Partikel, die größer als 2000µm, insbesondere im Wesentlichen keine Partikel, die größer sind als 500 µm, enthält.
21. Verwendung eines Kochschrittes für mindestens 30 Minuten, bevorzugt für mindestens 45 Minuten, mehr bevorzugt für mindestens 1 Stunde, noch mehr bevorzugt für mindestens 2 Stunden, insbesondere für mindestens 3 Stunden, zur Erhöhung des Gehaltes an Avenanthramiden, insbesondere Avenanthramid B, in einem Hafermehlprodukt.

Ferner wird die Erfindung auch anhand der nachfolgenden Ausführungsformen, auf die sie selbstverständlich nicht eingeschränkt ist, näher erläutert:
A1. Verfahren zur Herstellung eines Präparats aus Carica papaya-Früchten und Hafer (*Avena sativa*)-Mehl, umfassend:
   a) Aufschlämmen des Hafermehls in vorzugsweise kaltem Wasser (Bestandteil B);
   b) Erhitzen der Aufschlämmung unter Rühren auf Kochtemperatur;
   c) Kochen der Aufschlämmung unter Rühren, vorzugsweise für mindestens 30 Minuten, insbesondere für mindestens 3 Stunden;
   d) Zugeben eines Mus-Präparats aus Carica papaya-Früchten (Bestandteil A*) zur, vorzugsweise kochenden, Aufschlämmung, wobei Bestandteil A* erhältlich ist nach einem Verfahren, gekennzeichnet durch die folgenden Schritte:
      - Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
      - Abkühlen der gekochten Früchte oder zerkleinerten Früchte über einen Zeitraum von zumindest 30 min unter Sauerstoff-haltiger Atmosphäre, vorzugsweise unter Luftzufuhr und bei Raumtemperatur,
      - gegebenenfalls Zerkleinern, Mischen und Passieren der abgekühlten Früchte oder zerkleinerten Früchte bis zum Erhalt eines homogenen Muses,
   vorzugsweise dadurch gekennzeichnet, dass das Zugeben von Bestandteil A* unter Rühren und/oder bis zu einer Gesamtkonzentration von Bestandteil A* von 5%-50% (v/v), vorzugsweise 10%-40% (v/v), insbesondere 15%-25% (v/v) erfolgt.
A2. Verfahren nach Ausführungsform A1, ferner einen oder mehrere der folgenden Schritte umfassend:
   e) Zugeben von Wasser;
   f) Halten des Gemischs auf 70°C-90°C, insbesondere auf 75°C-85°C für mindestens 10 Minuten;
   g) Zugeben von Bindemittel(n), Aromastoff(en) und/oder Konservierungsmittel(n);
   h) Einstellen des pH-Werts durch Zugeben von Zitronensaftkonzentrat, Zitronensäure und/oder einem anderen genießbaren Säuerungsmittel auf 4 bis 6, vorzugsweise 4,5 bis 5,5.
A3. Präparat, erhältlich nach einem Verfahren gemäß Ausführungsform A1 oder A2, wobei das Hafermehl vorzugsweise als wässriges Kolloid vorliegt.
A4. Präparat aus Carica papaya-Früchten und Hafermehl, umfassend:
   A) Mus-Präparat aus Carica papaya-Früchten, erhältlich nach einem Verfahren, gekennzeichnet durch die folgenden Schritte:
      - Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
      - Abkühlen der gekochten Früchte oder zerkleinerten Früchte über einen Zeitraum von zumindest 30 min unter Sauerstoff-haltiger Atmosphäre, vorzugsweise bei Raumtemperatur,
      - gegebenenfalls Zerkleinern, Mischen und Passieren der abgekühlten Früchte oder zerkleinerten Früchte bis zum Erhalt eines homogenen Muses; und
   B) Hafermehl, vorzugsweise als wässriges Kolloid.
A5. Präparat nach Ausführungsformen A3 oder A4, ferner umfassend Zitronensaftkonzentrat, Konservierungsmittel, Speisestärke, Aromastoff(en) und/oder Wasser, gegebenenfalls ohne weitere Inhaltsstoffe.
A6. Präparat nach einer der Ausführungsformen A3 bis A5, dadurch gekennzeichnet, dass die Gesamtkonzentration (v/v) von Speisestärke und Konservierungsmittel unabhängig voneinander jeweils zwischen 0% und 2%, insbesondere zwischen 0.5% und 1.5%, liegt und gegebenenfalls dadurch gekennzeichnet, dass es pasteurisiert ist.
A7. Präparat nach einer der Ausführungsformen A3 bis A6, dadurch gekennzeichnet, dass der Wassergehalt (v/v) des Präparats zwischen 75% und 85%, insbesondere zwischen 78% und 82%, liegt.
A8. Präparat nach einer der Ausführungsformen A3 bis A7, dadurch gekennzeichnet, dass der pH-Wert des Präparats zwischen 4 und 6, insbesondere zwischen 4,5 und 5,5, liegt.
A9. Präparat nach einer der Ausführungsformen A3 bis A8, dadurch gekennzeichnet, dass die Viskosität des Präparats zwischen 5000 und 10000 cP, insbesondere zwischen 6000 und 8000 cP, liegt.
A10. Präparat nach einer der Ausführungsformen A3 bis A9, dadurch gekennzeichnet, dass beim Mus-Präparat aus Carica papaya-Früchten:
   - das Kochen mindestens 2h durchgeführt wird; und/oder
   - das Abkühlen mindestens 5h durchgeführt wird; und/oder
   - die Früchte vor dem Kochen geschält und entkernt werden; und/oder
   - die Früchte halbreif bis reif sind;
   und/oder dass das Mus-Präparat aus Carica papaya-Früchten pasteurisiert ist.
A11. Präparat nach einer der Ausführungsformen A3 bis A10, zur Anwendung in therapeutischen Verfahren.
A12. Präparat nach Ausführungsform A11, zur Anwendung in der Behandlung von Reiz- und/oder Entzündungszuständen der Haut oder Schleimhaut, ausgewählt aus:
   Dermatitis, insbesondere atopischer Dermatitis, Juckreiz, Rötung, trockener, rissiger, offener, nässender, verdickter, entzündeter Haut oder Schleimhaut, Sonnenbrand, Insektenstiche und Psoriasis,
   und dadurch gekennzeichnet, dass die Anwendung vorzugsweise auf Haut oder Schleimhaut erfolgt, insbesondere direkt am Ort der Reiz- und/oder Entzündungszustände.
A13. Präparat nach Ausführungsform A11, zur Anwendung in der Behandlung von Verdauungsstörungen, vorzugsweise ausgewählt aus der Gruppe bestehend aus chronische Obstipation, Flatulenz und Reizdarmsyndrom, dadurch gekennzeichnet, dass das Präparat oral verabreicht wird.
A14. Präparat nach Ausführungsform A11, zur Anwendung in der Behandlung von einem oder mehreren Krankheiten oder Zuständen, ausgewählt aus:
   - geschwächtes Immunsystem
   - Wunden, insbesondere bei Ulcus cruris,
   - Diabetes.
A15. Kosmetisches nicht-therapeutisches Verfahren, umfassend die Anwendung des Präparats nach einer der Ausführungsform A1 bis A10, vorzugsweise auf der Haut, zum Erhalt eines schöneren Hautbildes.

Die Erfindung wird anhand der nachfolgenden Figuren und Beispiele, auf die sie selbstverständlich nicht eingeschränkt ist, näher erläutert.

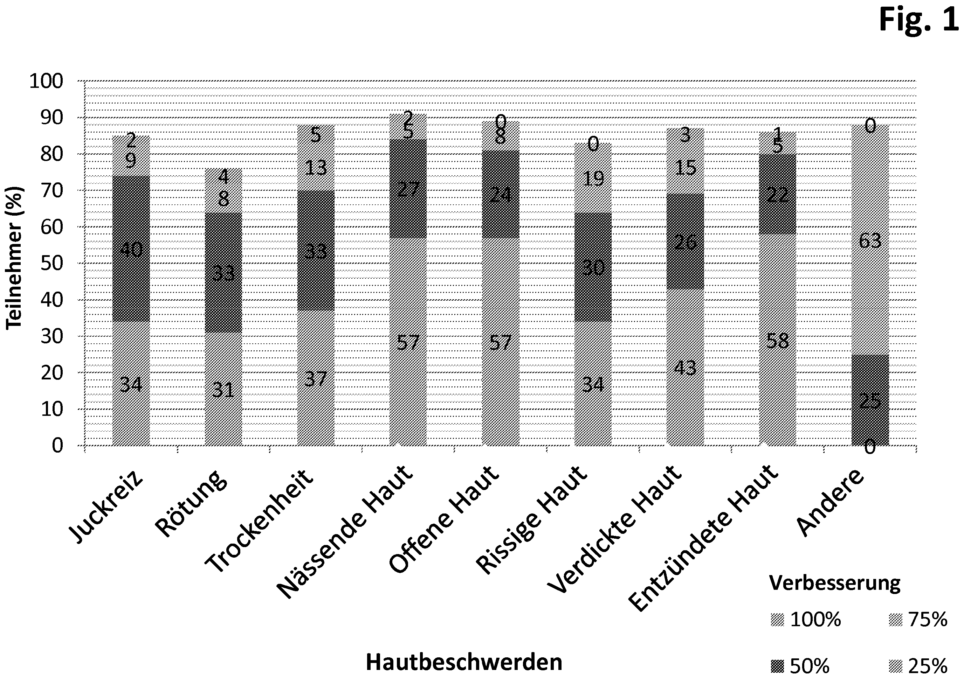

Fig. 1: Verbesserung bei verschiedenen Hautbeschwerden nach Anwendung des Präparats der vorliegenden Erfindung (Y-Achse: Prozent der Probanden, die mindestens 25% Verbesserung nach Anwendung aufwiesen), wobei zusätzlich zwischen 25%, 50%, 75% und 100% Verbesserung unterschieden wird.
Fig. 2: Avenanthramide-Konzentration (in ppm) in Bezug auf das jeweilige Trockengewicht von Hafermehl vor der Zubereitung ("H0"; d.h. vor dem erfindungsgemäßen Verfahren) und der Papaya-Hafer-Zubereitung ("HP1"; das heißt dem erfindungsgemäß Papaya-Hafer-Präparat, umfassend das spezielle Papaya-Mus-Präparat; HP1).
Fig. 3: Das Hafermehl-Papaya-Präparat (Probe A) zeigt einen dosisabhängigen, hemmenden Effekt auf die Freisetzung von Interleukin-8 (IL8) bei humanen epidermalen Keratinozyten (HEKa). "-" ist die Negativkontrolle (kein TNF-alpha), ansonsten beträgt die TNF-alpha-Konzentration 10ng/ml. OD: Optische Dichte.

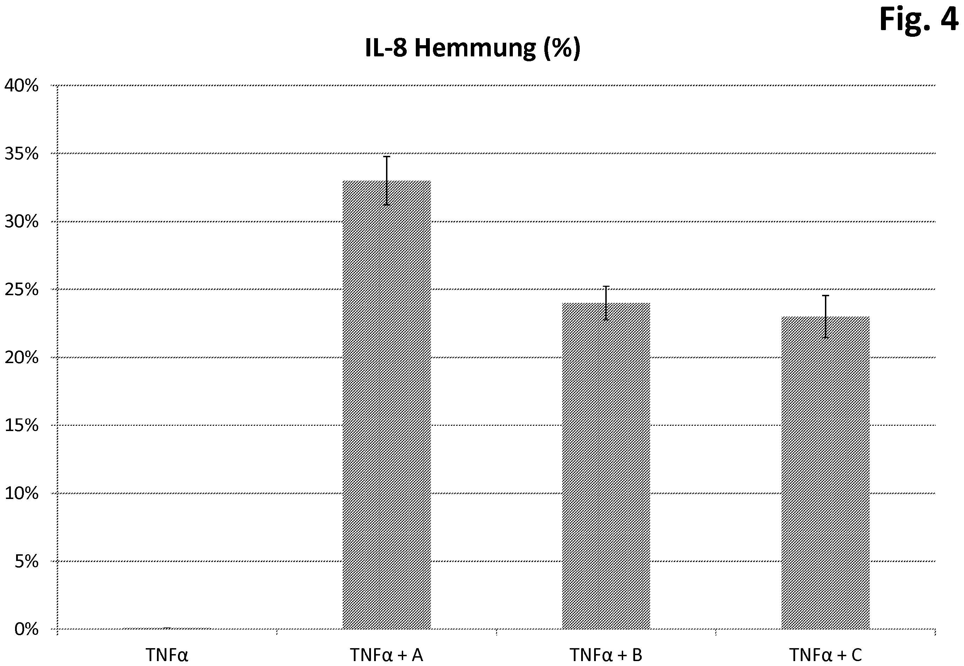

Fig. 4: Die Kombination von Hafermehl und Papaya im erfindungsgemäßen Präparat bewirkt überraschenderweise synergistisch eine Hemmung der IL8-Freisetzung bei humanen Darmepithelzellen (Zelllinie HT29-MTX-E12). Gezeigt ist die Verringerung der IL-8 Konzentration gegenüber der TNF-alpha-Kontrolle (10ng/ml). A: Präparat aus Hafermehl und Papaya-Mus (1mg/ml), B: Präparat aus Hafermehl (1mg/ml), C: Papaya-Mus (1mg/ml).

### Beispiele

### Beispiel 1

### Herstellung des erfindungsgemäßen Präparats

Bio-Hafermehl mit einem maximalen Partikeldurchmesser von 1000µm wird im doppelwandigen Kessel ausgestattet mit Umlauferhitzer in kaltem Wasser aufgeschlämmt (Bestandteil B). Unter ständigem Rühren wird die Aufschlämmung auf 100°C erhitzt und für drei Stunden bei 100°C gekocht und dadurch eingedickt. Der doppelwandige Kessel mit Umlauferhitzer sorgt für gleichmäßige Temperaturverteilung und schnelles Erreichen der gewünschten Temperatur, des weiteren kommt es durch das Umwälzverfahren zu einer Anreicherung des Sauerstoffs im Produkt.

Als Qualitätsmerkmal werden nun pH, Wassergehalt und Viskosität der Aufschlämmung bestimmt (und evtl. angepasst), mit den Sollparametern: pH 6-7 , Wassergehalt 85-90% und Viskosität 25000-30000 cP.

Dann wird das Papaya-Fruchtmus (Bestandteil A, bekannt aus der WO 2004/047850 A1 und auch in der vorliegenden Anmeldung beschrieben) bis zu einer Gesamtkonzentration von 10-40% (v/v) zur kochenden Aufschlämmung unter Rühren zugegeben, worauf das Gemisch unter Rühren auf 75-85°C erhitzt bzw. gehalten wird, für 5-15 Minuten.

Als Qualitätsmerkmal werden nun pH, Wassergehalt und Viskosität des Gemisches bestimmt (und evtl. angepasst), mit den Sollparametern: pH 4,5-5,5 , Wassergehalt 75%-95% und Viskosität 3000-10000 cP, wobei der pH-Wert mit Bio-Zitronensaftkonzentrat eingestellt wird, das Gemisch wird dabei immer auf 80°C gehalten.

Schließlich wird das Gemisch (nun das erfindungsgemäße Präparat) mit einer Temperatur von 70-90°C in aseptische Beutel abgefüllt.

### Beispiel 2

### Test des erfindungsgemäßen Präparats an menschlichen Patienten

Das Präparat der vorliegenden Erfindung wurde in einer Pilotstudie mit freiwilligen Probanden getestet, wobei das Präparat auf der Haut der Probanden angewendet wurde. Die Stärke verschiedener Hautbeschwerden wurde vor und nach der Anwendung von den Probanden beurteilt und die Verbesserung der Beschwerden nach der Testphase ausgewertet (Fig. 1). Es zeigte sich eine wesentliche Verbesserung (bei ca. 85% der Probanden) durch die Anwendung der Papaya-Hafer-Zubereitung bei den Hautbeschwerden Juckreiz, Rötung, trockener, rissiger/offener, nässender, verdickter und/oder entzündeter Haut.

### Beispiel 3

### Test des erfindungsgemäßen Präparats

Die entzündungshemmende Wirkung des Präparats auf Haut und/oder Schleimhaut wird durch die Ergebnisse einer Studie zum Entzündungsmarker alpha-1 Antitrypsin untermauert, welcher bei Schleimhautreizungen und/oder Schleimhautentzündungen verstärkt gebildet und nachgewiesen werden kann. Durch die Anwendung des Präparats kommt es zu einer Reduktion dieses Entzündungsmarkers, was auf eine Beruhigung und ein Abheilen der Schleimhautveränderungen hinweist.

### Beispiel 4

### Messung der Avenanthramid-Konzentration

Mittels eines externen Avenanthramid B Standards (Sigma) wurde durch HPLC-Analyse und UV-Detektion die Avenanthramid B-Konzentrationen in vier Proben gemessen:
P0... Papaya vor Zubereitung zum Fruchtmus
P1... Papaya-Fruchtmus (das spezielle Papaya-Fruchtmus)
H0... Hafermehl vor Zubereitung
H1... Hafermehl nach Zubereitung (das erfindungsgemäß Präparat, ohne jegliche Papaya-Komponente)

HP1...Hafermehl+Papaya-Fruchtmus nach Zubereitung (das erfindungsgemäß Präparat umfassend das spezielle Papaya-Fruchtmus). Verhältnis Trockenmasse Hafermehl:Papayafruchtmus ist 80:20.

Avenanthramid B ist "5-Hydroxy-2-{[(2*E*)-3-(4-hydroxy-3-methoxyphenyl)-1-oxo-2-propenyl]amino}benzoic acid, *N*-[4'-Hydroxy-3'-methoxy-(*E*)-cinnamoyl]-5-hydroxyanthranilic "

### Probenvorbereitung:

Die Proben wurden mit 20% v/v Ethanol im Ultraschallbad 2 mal für 15 min extrahiert und anschliessed bei 14500 rpm zentrifugiert. Der Überstand wurde direkt für die HPLC Analyse verwendet.

Von der Referenzsubstanz Avenanthramid B (Sigma) wurde 0.44 mg in Ethanol 96% gelöst und anschliessend mit 20% Ethanol auf 10.0 ml verdünnt.

### HPLC Methode

- Säule Atlantis T3 3.5 µm 4.6 x 150 mm
- Laufmittel
A: Acetonitril
B: 1% Phosphorsäure

- Gradient

| Time | | A% | B% |
|---|---|---|---|
| 0 | | 20 | 80 |
| 20 | | 60 | 40 |
| 30 | | 100 | 0 |
| 35 | | 100 | 0 |
| 36 | | 20 | 80 |

- Auto sampler Zeit 41 min
- Injektionsvol. Probe: 100 µl
Referenzlösung: 5 µl
- UV-Detektion 340 nm
- Fluss 0.5 ml/min

### Resultate

Es konnte in den Haferproben (H0, H1, HP1) Avenanthramid B nachgewiesen werden (Tab. 1; Abb. 1-3). Es zeigte sich, dass in H1 (erfindungsgemäßes Präparat) ein höheren Avenanthramid Gehalt auftrat als H0 (Hafermehl vor der Zubereitung). In P0 und P1 konnte Avenanthramid B wie erwartet nicht nachgewiesen werden.

**Tab. 1: Avenanthramidgehalte in Bezug auf die Trockenmasse**

| **Muster** | **Trockenmasse [%]** | **Avenanthramide B bezogen auf Gesamttrockenmasse [ppm]** |
|---|---|---|
| P1 | 13.9 | Nicht nachweisbar |
| P0 | 9.7 | Nicht nachweisbar |
| H0 | 92.8 | 894 |
| H1 | 13.1 | 2069 |
| HP1 | 13.9 | 1849 |

Die Nachweisgrenze der Methode betrug 0.044 ppm, die Bestimmungsgrenze 0.1 ppm.

Die gemessenen Konzentrationen von Avenanthramiden in den hier untersuchten Haferzubereitungen sind ausreichend um bei einer topischen Applikation die oben beschriebenen Wirkungen zu zeigen.

Der Haferanteil in HP1 beträgt nur 80% der Gesamttrockenmasse. Somit wäre eigentlich anhand der Trockenmassenverhältnisse zwischen Hafer- und Papayakomponente (durch den Verdünnungseffekt) eine Avenanthramid-Konzentration von 1655ppm statt 1849ppm zu erwarten gewesen. Normalisiert auf die Hafertrockenmasse ist die Avenanthramid-Konzentration in HP1 also 2311ppm, was einer überraschenden Steigerung von mehr als 10% im Vergleich zu den 2069ppm von HP1 entspricht.

Somit ist erwiesen, dass die Papaya-Komponente einen überraschenden vorteilhaften Effekt in Bezug auf die Avenanthramid-Konzentration der Haferkomponente ausübt.

### Beispiel 5 - Entzündungshemmende Wirkung des erfindungsgemäßen Präparats bei menschlichen Hautepithelzellen

Die entzündungshemmende Wirkung des Hafermehl-Papaya-Präparats wurde anhand der Wirkung des Präparats auf die Freisetzung eines pro-inflammatorischen Cytokins, Interleukin-8 (IL-8), bei Tumor-Nekrose-Faktor-alpha-(TNF-alpha)-stimulierten Hautepithelzell-Kulturen untersucht. Üblicherweise löst Stimulation von Hautepithelzellen mit TNF-alpha die Freisetzung von IL-8 durch die Hautepithelzellen aus, womit wiederum eine Entzündungsreaktion ausgelöst oder verstärkt wird.

### Probe:

Präparat aus Hafermehl und Papaya-Mus, gemäß der vorliegenden Erfindung; Kochzeit: 3 Stunden, mittlerer Hafermehlpartikeldurchmesser: 1000µm, Anteil Papayamus (v/v): ca. 20%, Gesamtwassergehalt ca. 80% (v/v), pH 5. Die Probe wird in diesem Beispiel als "Probe A" oder "A" bezeichnet.

### Materialien:

- Humane epidermale Keratinozyten, adult (HEKa), Gibco®
- "Human IL-8 ELISA development kit", PromoKine
- "Cell Counting Kit-8", Dojindo Molecular Technologies
- Menschliches rekombinantes TNF-alpha, PromoKine
- Dexamethason wasserlöslich, Sigma

### Methoden:

Cytokin-Assay: Probe A wurde jeweils bis zur angegebenen Konzentration (0-0,25mg/ml) zu mehreren konfluenten Zellkulturen in Zellkulturmedium zugegeben. Für 30 Minuten wurden die Zellkulturen nun im Inkubator (37°C, 5% CO₂) vorinkubiert. Danach wurde TNF-alpha bis zu einer Konzentration von 10ng/ml hinzugefügt und für weitere 24 Stunden inkubiert. Zusätzlich wurde eine Zellkultur nicht mit TNF-alpha inkubiert (Negativkontrolle). Danach wurde die jeweils freigesetzte Konzentration an IL-8 mit dem ""Human IL-8 ELISA development kit" untersucht. Ferner wurde die grundsätzliche Funktionsweise des Assays mit dem anti-inflammatorischen Wirkstoff Dexamethason (als Positivkontrolle) sichergestellt.

Cytotoxizitäts-Assay: Probe A wurde auch auf eine etwaige Cytotoxizität hin untersucht. Dafür wurden Zellkulturen wie im Cytokin-Assay behandelt, jedoch wurde anstelle der IL-8 Konzentration die Anzahl der noch lebenden Zellen mittels des "Cell-Counting Kits-8", einem Tetrazolium-Salz-basierten kolorimetrischen Test (ähnlich dem "MTT-Test"), erhoben.

### Resultate:

Probe A war bei keiner der untersuchten Konzentrationen cytotoxisch. Es zeigte sich ein dosisabhängiger inhibitorischer Effekt von Probe A auf die IL-8-Freisetzung bei menschlichen epidermalen Keratinozyten (Fig. 3). Damit ist eine entzündungshemmende Wirkung des erfindungsgemäßen Präparats bei Hautzellen nachgewiesen.

### Beispiel 6 - Entzündungshemmende Wirkung des erfindungsgemäßen Präparats bei menschlichen Darmepithelzellen (Vergleichsbeispiel)

Die entzündungshemmende Wirkung des Präparats wurde anhand der Wirkung des Präparats auf die Freisetzung eines pro-inflammatorischen Cytokins, Interleukin-8 (IL-8), bei Tumor-Nekrose-Faktor-alpha-(TNF-alpha)-stimulierten Darmepithelzell-Kulturen untersucht. Üblicherweise löst Stimulation von Darmepithelzellen mit TNF-alpha die Freisetzung von IL-8 durch die Darmepithelzellen aus, womit wiederum eine Entzündungsreaktion ausgelöst oder verstärkt wird.

### Proben:

- Probe A: Präparat aus Hafermehl und Papaya-Mus, gemäß der vorliegenden Erfindung; Kochzeit: 3 Stunden, mittlerer Hafermehlpartikeldurchmesser: 1000µm, Anteil Papayamus (v/v): ca. 20%, Gesamtwassergehalt ca. 80% (v/v), pH 5.
- Probe B: Präparat aus Hafermehl, hergestellt gemäß der vorliegenden Erfindung; Kochzeit: 3 Stunden, mittlerer Hafermehlpartikeldurchmesser: 1000µm, Anteil Papayamus (v/v): 0%, Gesamtwassergehalt ca. 80% (v/v), pH 5.
- Probe C: Papaya-Mus, wie zu Probe A hinzu gegegeben.

### Materialien:

- Humane Darmepithelzellen, Zelllinie HT29-MTX-E12 (ECACC)
- "Human IL-8 ELISA development kit", PromoKine
- "Cell Counting Kit-8", Dojindo Molecular Technologies
- Menschliches rekombinantes TNF-alpha, PromoKine
- Dexamethason wasserlöslich, Sigma

### Methoden:

Cytokin-Assay: Proben A, B und C wurde jeweils bis zur Konzentration von 1mg/ml zu mehreren konfluenten Zellkulturen in Zellkulturmedium zugegeben. Für 30 Minuten wurden die Zellkulturen nun im Inkubator (37°C, 5% CO₂) vorinkubiert. Danach wurde TNF-alpha bis zu einer Konzentration von 10ng/ml hinzugefügt und für weitere 24 Stunden inkubiert. Zusätzlich wurde eine Zellkultur nicht mit TNF-alpha inkubiert (Negativkontrolle). Danach wurde die jeweils freigesetzte Konzentration an IL-8 mit dem "Human IL-8 ELISA development kit" untersucht. Ferner wurde die grundsätzliche Funktionsweise des Assays mit dem anti-inflammatorischen Wirkstoff Dexamethason (als Positivkontrolle) sichergestellt.

Cytotoxizitäts-Assay: Proben A, B und C wurde auch auf eine etwaige Cytotoxizität hin untersucht. Dafür wurden Zellkulturen wie im Cytokin-Assay behandelt, jedoch wurde anstelle der IL-8 Konzentration die Anzahl der noch lebenden Zellen mittels des "Cell-Counting Kits-8", einem Tetrazolium-Salz-basierten kolorimetrischen Test (ähnlich dem "MTT-Test"), erhoben.

### Resultate:

Die Proben A, B und C waren bei keiner der untersuchten Konzentrationen cytotoxisch. Es zeigte sich durch die erfindungsgemäße Kombination von Hafermehl und Papaya ein synergistischer hemmender Effekt auf die IL-8-Freisetzung (Fig. 4) .

## Patentansprüche

1. Verfahren zur Herstellung eines Präparats aus Hafer(Avena *sativa*)-Mehl und *Carica papaya*-Früchten, umfassend:
a) Aufschlämmen des Hafermehls in vorzugsweise kaltem Wasser;
b) Erhitzen der Aufschlämmung, vorzugsweise unter Rühren, auf Kochtemperatur;
c) Kochen der Aufschlämmung unter Rühren für mindestens 30 Minuten, bevorzugt für mindestens 45 Minuten, mehr bevorzugt für mindestens 1 Stunde, noch mehr bevorzugt für mindestens 2 Stunden, insbesondere für mindestens 3 Stunden;
d) Zugeben eines Präparats aus *Carica* papaya-Früchten (Bestandteil A) zur, vorzugsweise kochenden, Aufschlämmung (Bestandteil B);
wobei das Hafermehl einen durchschnittlichen Partikeldurchmesser von weniger als 2000µm, bevorzugt weniger als 1500µm, mehr bevorzugt weniger als 1000µm, noch mehr bevorzugt weniger als 500µm und insbesondere weniger als 350µm aufweist.

2. Verfahren nach Anspruch 1, wobei Bestandteil A in Schritt d) ein Mus-Präparat aus *Carica* papaya-Früchten ist, und/oder wobei das Verfahren **dadurch gekennzeichnet ist, dass** das Zugeben von Bestandteil A unter Rühren und/oder bis zu einer Gesamtkonzentration von Bestandteil A von 5%-50% (v/v), vorzugsweise 10%-40% (v/v), insbesondere 15%-25% (v/v) erfolgt.

3. Verfahren nach Anspruch 1 oder 2, wobei Bestandteil A ein Mus-Präparat aus *Carica* papaya-Früchten ist, erhältlich nach einem Verfahren, **gekennzeichnet durch** die folgenden Schritte:
- Kochen der Früchte oder zerkleinerten Früchte, insbesondere der Früchte in gesiebter Form, für mindestens 30 min bei Normaldruck, gegebenenfalls mit dem mindestens 2-fachen Volumen einer wässerigen Lösung,
- Abkühlen der gekochten Früchte oder zerkleinerten Früchte über einen Zeitraum von zumindest 30 min unter Sauerstoff-haltiger Atmosphäre, vorzugsweise unter Luftzufuhr und bei Raumtemperatur,
- gegebenenfalls Zerkleinern, Mischen und Passieren der abgekühlten Früchte oder zerkleinerten Früchte bis zum Erhalt eines homogenen Muses.

4. Verfahren nach einem der Ansprüche 1 bis 3, ferner einen oder mehrere der folgenden Schritte umfassend:
e) Zugeben von Wasser;
f) Halten des Gemischs auf 70°C-90°C, insbesondere auf 75°C-85°C für mindestens 10 Minuten;
g) Zugeben von Bindemittel(n), Aromastoff(en) und/oder Konservierungsmittel(n);
h) Einstellen des pH-Werts durch Zugeben von Zitronensaftkonzentrat, Zitronensäure und/oder einem anderen genießbaren Säuerungsmittel auf 4 bis 6, vorzugsweise 4,5 bis 5,5.

5. Präparat, erhältlich nach einem Verfahren gemäß einem der Ansprüche 1 bis 4, vorzugsweise wobei das Präparat durch das Kochen hygienisch ist, und/oder vorzugsweise wobei das Hafermehl als wässriges Kolloid vorliegt.

6. Präparat nach Anspruch 5, wobei die Konzentration der Avenanthramide in Bezug auf die Gesamttrockenmasse des Präparats mindestens 500ppm, bevorzugt mindestens 1000ppm, mehr bevorzugt mindestens 1250ppm, noch mehr bevorzugt mindestens 1500ppm, insbesondere mindestens 1750ppm beträgt; vorzugsweise wobei Avenanthramid B einen Anteil an besagten Avenanthramiden hat, bevorzugt von mindestens 5%, mehr bevorzugt von mindestens 10%, noch mehr bevorzugt von mindestens 20%, insbesondere von mehr als 50% oder gar mehr als 75%.

7. Präparat nach Anspruch 5, wobei die Konzentration der Avenanthramid B in Bezug auf die Gesamttrockenmasse des Hafermehls im Präparat mindestens 1000ppm, bevorzugt mindestens 1500ppm, mehr bevorzugt mindestens 1750ppm, noch mehr bevorzugt mindestens 2000ppm, insbesondere mindestens 2250ppm beträgt.

8. Präparat nach einem der Ansprüche 5 bis 7, ferner umfassend Zitronensaftkonzentrat, Konservierungsmittel, Speisestärke, Aromastoff(en) und/oder Wasser, gegebenenfalls ohne weitere Inhaltsstoffe.

9. Präparat nach einem der Ansprüche 5 bis 8, **dadurch gekennzeichnet, dass** der Wassergehalt (v/v) des Präparats zwischen 75% und 95%, vorzugsweise zwischen 80% und 93%, insbesondere zwischen 85% und 90%, liegt; und/oder **dadurch gekennzeichnet, dass** der pH-Wert des Präparats zwischen 4 und 6, insbesondere zwischen 4,5 und 5,5, liegt; und/oder **dadurch gekennzeichnet, dass** die Viskosität des Präparats zwischen 3000 und 10000 cP, insbesondere zwischen 4000 und 8000 cP, liegt.

10. Präparat nach einem der Ansprüche 5 bis 9, beinhaltend das Mus-Präparat aus *Carica* papaya-Früchten erhältlich nach einem Verfahren, wobei das Verfahren zusätzlich **dadurch gekennzeichnet ist, dass**:
- das Kochen mindestens 2h durchgeführt wird; und/oder
- das Abkühlen mindestens 5h durchgeführt wird; und/oder
- die Früchte vor dem Kochen geschält und entkernt werden; und/oder
- die Früchte halbreif bis reif sind;
und/oder dass das Mus-Präparat aus *Carica* papaya-Früchten pasteurisiert ist.

11. Präparat nach einem der Ansprüche 5 bis 10, zur Anwendung in therapeutischen Verfahren.

12. Präparat zur Anwendung nach Anspruch 11, zur Behandlung von Reiz- und/oder Entzündungszuständen der Haut oder Schleimhaut, ausgewählt aus:
Dermatitis, insbesondere atopischer Dermatitis, Juckreiz, Rötung, trockener, rissiger, offener, nässender, verdickter, entzündeter Haut oder Schleimhaut, Sonnenbrand, Insektenstiche und Psoriasis,
**dadurch gekennzeichnet, dass** die Anwendung vorzugsweise auf Haut oder Schleimhaut erfolgt, insbesondere direkt am Ort der Reiz- und/oder Entzündungszustände.

13. Präparat zur Anwendung nach Anspruch 11, zur Behandlung von Verdauungsstörungen, vorzugsweise ausgewählt aus der Gruppe bestehend aus chronischer Obstipation, Flatulenz, Gastritis, Sodbrennen und Reizdarmsyndrom, **dadurch gekennzeichnet, dass** das Präparat oral verabreicht wird.

14. Kosmetisches nicht-therapeutisches Verfahren, umfassend die Anwendung des Präparats nach einem der Ansprüche 5 bis 10, vorzugsweise auf der Haut, zum Erhalt eines schöneren Hautbildes.

## Claims

1. Method for making a preparation of oat (*Avena sativa*) flour and *Carica papaya* fruit, comprising:
a) slurrying the oat flour in preferably cold water;
b) heating the slurry, preferably while stirring, to boiling point;
c) boiling the slurry while stirring for at least 30 minutes, preferably for at least 45 minutes, more preferably for at least 1 hour, even more preferably for at least 2 hours, in particular for at least 3 hours;
d) adding a preparation of *Carica papaya* fruit (component A) to the preferably boiling slurry (component B);
wherein the oat flour has an average particle diameter of less than 2000 µm, preferably less than 1500 µm, more preferably less than 1000 µm, even more preferably less than 500 µm, and in particular less than 350 µm.

2. Method according to claim 1, component A in step d) being a puree preparation of *Carica papaya* fruit, and/or the method being **characterised in that** component A is added while stirring and/or up to a total concentration of component A of 5%-50% (v/v), preferably 10%-40% (v/v), in particular 15%-25% (v/v).

3. Method according to either claim 1 or claim 2, component A being a puree preparation of *Carica papaya* fruit that can be obtained using a method **characterised by** the following steps:
- boiling the fruit or crushed fruit, in particular the fruit in sieved form, for at least 30 minutes at normal pressure, optionally with at least twice the volume of an aqueous solution,
- cooling the boiled fruit or crushed fruit for a period of at least 30 minutes in an atmosphere containing oxygen, preferably with an air supply present and at room temperature,
- optionally crushing, mixing, and straining the cooled fruit or crushed fruit until a homogeneous puree is obtained.

4. Method according to any of claims 1 to 3, further comprising one or more of the following steps:
e) adding water;
f) maintaining the mixture at 70°C-90°C., in particular at 75°C-85°C, for at least 10 minutes;
g) adding binder(s), flavouring(s) and/or preservative(s);
h) adjusting the pH to 4 to 6, preferably 4.5 to 5.5, by adding lemon juice concentrate, citric acid and/or another edible acidifier.

5. Preparation that can be obtained using a method according to any of claims 1 to 4, wherein the preparation is preferably sanitary as a result of the boiling and/or wherein the oat flour is preferably present as an aqueous colloid.

6. Preparation according to claim 5, wherein the concentration of avenanthramides with respect to the total dry mass of the preparation is at least 500 ppm, preferably at least 1000 ppm, more preferably at least 1250 ppm, even more preferably at least 1500 ppm, in particular at least 1750 ppm; wherein avenanthramide B is preferably a proportion of said avenanthramides, preferably at least 5%, more preferably at least 10%, even more preferably at least 20%, in particular more than 50% or even more than 75%.

7. Preparation according to claim 5, wherein the concentration of avenanthramide B with respect to the total dry mass of the oat flour in the preparation is at least 1000 ppm, preferably at least 1500 ppm, more preferably at least 1750 ppm, even more preferably at least 2000 ppm, in particular at least 2250 ppm.

8. Preparation according to any of claims 5 to 7, further comprising lemon juice concentrate, preservatives, food starch, flavouring(s) and/or water, optionally without further ingredients.

9. Preparation according to any of claims 5 to 8, **characterised in that** the water content (v/v) of the preparation is between 75% and 95%, preferably between 80% and 93%, in particular between 85% and 90%; and/or **characterised in that** the pH of the preparation is between 4 and 6, in particular between 4.5 and 5.5; and/or **characterised in that** the viscosity of the preparation is between 3000 and 10000 cP, in particular between 4000 and 8000 cP.

10. Preparation according to any of claims 5 to 9, containing the puree preparation of *Carica papaya* fruit that can be obtained using a method, the method being further **characterised in that**:
- the boiling is carried out for at least 2 hours; and/or
- the cooling is carried out for at least 5 hours; and/or
- the fruit is peeled and cored before the boiling; and/or
- the fruit is semi-ripe to ripe;
and/or **in that** the puree preparation of *Carica papaya* fruit is pasteurised.

11. Preparation according to any of claims 5 to 10, for use in therapeutic methods.

12. Preparation for use according to claim 11, for treating irritated and/or inflammatory conditions of the skin or mucosa, selected from:
dermatitis, in particular atopic dermatitis, itching, redness, dry, cracked, open, weeping, thickened, inflamed skin or mucosa, sunburn, insect bites and psoriasis,
**characterised in that** the preparation is preferably applied to skin or mucosa, in particular directly at the site of the irritated and/or inflammatory conditions.

13. Preparation for use according to claim 11, for treating digestive disorders, preferably selected from the group consisting of chronic constipation, flatulence, gastritis, heartburn and irritable bowel syndrome, **characterised in that** the preparation is administered orally.

14. Non-therapeutic cosmetic method, comprising the application of the preparation according to any of claims 5 to 10, preferably to the skin, in order to achieve an improved complexion.

## Revendications

1. Procédé de fabrication d'une préparation de farine d'avoine (*Avena sativa*) et de fruits de *Carica papaya,* comprenant :
a) la mise en suspension de la farine d'avoine dans de l'eau de préférence froide ;
b) le chauffage de la suspension, de préférence sous agitation, jusqu'à la température d'ébullition ;
c) la cuisson de la suspension sous agitation pendant au moins 30 minutes, de préférence pendant au moins 45 minutes, plus préférentiellement pendant au moins 1 heure, d'une manière encore plus préférée pendant au moins 2 heures, en particulier pendant au moins 3 heures ;
d) l'addition d'une préparation de fruits de *Carica papaya* (Constituant A) à la suspension (Constituant B), de préférence en ébullition ;
dans lequel la farine d'avoine présente une granulométrie moyenne inférieure à 2000 µm, de préférence inférieure à 1500 µm, plus préférentiellement inférieure à 1000 µm, d'une manière encore plus préférée inférieure à 500 µm et en particulier inférieure à 350 µm.

2. Procédé selon la revendication 1, dans lequel le Constituant A de l'étape d) est une préparation de purée de fruits de *Carica papaya,* et/ou le procédé étant **caractérisé en ce que** l'addition du Constituant A s'effectue sous agitation et/ou jusqu'à une concentration totale du Constituant A de 5 % à 50 % (v/v), de préférence de 10 % à 40 % (v/v), en particulier de 15 % à 25 % (v/v).

3. Procédé selon la revendication 1 ou 2, dans lequel le Constituant A est une préparation de purée de fruits de *Carica papaya,* pouvant être obtenue par un procédé **caractérisé par** les étapes suivantes :
- cuisson des fruits ou des fruits broyés, en particulier des fruits sous forme tamisée, pendant au moins 30 min sous la pression normale, éventuellement avec un volume au moins double d'une solution aqueuse,
- refroidissement des fruits cuits ou des fruits broyés sur un laps de temps d'au moins 30 min sous atmosphère contenant de l'oxygène, de préférence sous apport d'air et à la température ambiante,
- éventuellement, broyage, mélange et moulinage des fruits refroidis ou des fruits broyés, jusqu'à obtention d'une purée homogène.

4. Procédé selon l'une des revendications 1 à 3, comprenant en outre une ou plusieurs des étapes suivantes :
e) addition d'eau ;
f) maintien du mélange à 70 °C à 90 °C, en particulier à 75 °C à 85 °C, pendant au moins 10 minutes ;
g) addition d'un ou plusieurs liants, aromatisants et/ou conservateurs ;
h) ajustement du pH, par addition d'un concentré de jus de citron, d'acide citrique et/ou d'un autre acidifiant alimentaire, à 4 à 6, de préférence à 4,5 à 5,5.

5. Préparation pouvant être obtenue par un procédé selon l'une des revendications 1 à 4, de préférence la préparation étant hygiénique sous l'effet de la cuisson, et/ou de préférence la farine d'avoine se présentant sous forme d'un colloïde aqueux.

6. Préparation selon la revendication 5, la concentration des avénanthramides étant, par rapport à la masse sèche totale de la préparation, d'au moins 500 ppm, de préférence d'au moins 1000 ppm, plus préférentiellement d'au moins 1250 ppm, d'une manière encore plus préférée d'au moins 1500 ppm, en particulier d'au moins 1750 ppm ;
de préférence l'avénanthramide B étant présent, par rapport aux avénanthramides mentionnés, de préférence en une quantité d'au moins 5 %, plus préférentiellement d'au moins 10 %, d'une manière encore plus préférée d'au moins 20 %, en particulier supérieure à 50 % voire supérieure à 75 %.

7. Préparation selon la revendication 5, la concentration de l'avénanthramide B étant, par rapport à la masse sèche totale de la farine d'avoine dans la préparation, d'au moins 1000 ppm, de préférence d'au moins 1500 ppm, plus préférentiellement d'au moins 1750 ppm, d'une manière encore plus préférée d'au moins 2000 ppm, en particulier d'au moins 2250 ppm.

8. Préparation selon l'une des revendications 5 à 7, comprenant en outre un concentré de jus de citron, un conservateur, de la fécule, un ou plusieurs aromatisants et/ou de l'eau, éventuellement sans autres constituants.

9. Préparation selon l'une des revendications 5 à 8, **caractérisée en ce que** la teneur en eau (v/v) de la préparation est comprise entre 75 % et 95 %, de préférence entre 80 et 93 %, en particulier entre 85 et 90 % ; et/ou
**caractérisée en ce que** le pH de la préparation est compris entre 4 et 6, en particulier entre 4,5 et 5,5 ; et/ou
**caractérisée en ce que** la viscosité de la préparation est comprise entre 3000 et 10 000 cP, en particulier entre 4000 et 8000 cP.

10. Préparation selon l'une des revendications 5 à 9, contenant la préparation de purée de fruits de *Carica papaya,* pouvant être obtenue par un procédé qui est **caractérisé en outre en ce que** :
- la cuisson est effectuée pendant au moins 2 h ; et/ou
- le refroidissement est effectué pendant au moins 5 h ; et/ou
- les fruits sont pelés et dénoyautés avant la cuisson ; et/ou
- les fruits sont mi-mûrs à mûrs ;
et/ou que la préparation de purée de fruits de *Carica papaya* est pasteurisée.

11. Préparation selon l'une des revendications 5 à 10, pour une utilisation dans des procédures thérapeutiques.

12. Préparation pour l'utilisation selon la revendication 11, pour le traitement d'états d'irritation et/ou inflammatoires de la peau ou de la muqueuse, choisis parmi :
la dermatite, en particulier la dermatite atopique, les démangeaisons, les rougeurs, la peau ou la muqueuse sèche, crevassée, ouverte, suintante, épaissie, enflammée, les coups de soleil, les piqûres d'insectes et le psoriasis,
**caractérisée en ce que** l'utilisation a lieu de préférence sur la peau ou la muqueuse, en particulier directement à l'endroit des états d'irritation et/ou inflammatoires.

13. Préparation pour l'utilisation selon la revendication 11 pour le traitement des troubles de la digestion, choisis de préférence dans le groupe consistant en la constipation chronique, les flatulences, la gastrite, les brûlures d'estomac et le syndrome du côlon irritable, **caractérisée en ce que** la préparation est administrée par voie orale.

14. Procédure cosmétique non-thérapeutique, comprenant l'utilisation de la préparation selon l'une des revendications 5 à 10, de préférence sur la peau, pour obtenir un teint plus beau.
